(19) Europäisches Patentamt
European Patent Office
Office européen des brevets

(11) **EP 2 437 039 A2**

(12) **EUROPEAN PATENT APPLICATION**

(43) Date of publication:
**04.04.2012 Bulletin 2012/14**

(51) Int Cl.:
*G01K 1/14* (2006.01)  *G01K 3/06* (2006.01)
*G01K 13/00* (2006.01)

(21) Application number: **11275115.1**

(22) Date of filing: **28.09.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.09.2010 US 895210**

(71) Applicant: **Medisim Ltd.**
**90850 Neve Ilan (IL)**

(72) Inventor: **Yarden, Moshe**
**Mevaseret Zion (IL)**

(74) Representative: **Evens, Paul Jonathan**
**Maguire Boss**
**24 East Street**
**St. Ives**
**Cambridgeshire PE27 5PD (GB)**

(54) **Ergonomic hand held thermometer**

(57)     A thermometer (100) useful for measuring the temperature of a patient, the thermometer including a housing (102), at least one temperature sensor assembly (130) including a plurality of external patient skin contacting surfaces (138), and a mounting element (134) for mounting the at least one temperature sensor assembly (130) on the housing (102) and being operative for mutually independently mounting each of the plurality of external patient skin contacting surfaces (138) to be independently arrangeable in a spatial orientation which may be independently different from the spatial orientation of the external patient skin contacting surface of each other of the plurality of external patient skin contacting surfaces.

FIG. 1

EP 2 437 039 A2

**Description**

FIELD OF THE INVENTION

The present invention relates to apparatus for measurement of temperature of a person.

BACKGROUND OF THE INVENTION

The following publications are believed to represent the current state of the art:

U.S. Patent Nos. 2,658,390, 3,273,395, 3,282,106, 3,374,354, 3,392,282, 3,491,596, 3,581,570, 3,597,976, 3,614,892, 3,719,838, 3,777,568, 3,781,837, 3,933,045, 4,005,605, 4,133,700, 4,141,149, 4,198861, 4,204,120, 4,302971, 4,317,998, 4,372,690, 4,420,265, 4,428,382, 4,456390, 4,4 94881, 4,566,808, 4,602642, 4,623,266, 4,626,686, 4,634,294, 4,636,091, 4,691713, 4,722612, 4,726,688, 4,784149, 4,790,324, 4,797,840, 4,831,258, 4,843,577, 4,846583, 4,895164, 4,932,789, 4,993419, 5,012,813, 5,017,019, 5,018,872, 5,024,533, 5,050612, 5,056929, 5,150,969, 5,159936, 5,167,235, 5,178,464, 5,187,943, 5,199,436, 5,229612, 5,232284, 5,271,407, 5,325863, 5,333,784, 5,368,038, 5,381,796, 5,441,476, 5,445158, 5,469855, RE35,554, 5,645349, 5,653,238, 5,695,283, 5,743,644, 5,816,706, 5,818044, 5,872362, 5,874,736, 5,893833, 5,924,996, 5,924,998, 6,047,205, 6,056,435, 6,059452, 6,086247, 6,090,050, 6,220750, 6,280,397, 6,292,685, 6,299,347, 6,315,719, 6,416471, 6,447460, 6,499,877, 6,547745, 6,646,567, 6,709,154, 6,847,913, 6,890,096, 6,898457, 6,929611, 6,932,775, 6,963772, 7,213,969, 7,340,293, 7,346,386, 7,479,116, 7,597,668 and 7,625,117;

U.S. Published Patent Application Nos. 2001/0044588, 2001/0047127, 2002/0143257, 2003/0149349, 2003/0173408, 2004/0170216, 2005/0245839, 2006/0047218, 2006/0161074, 2006/0173375, 2006/0264730, 2007/0100666, 2008/0008225 and 2009/0299682;

Foreign Patent Documents DE 19 14 468; EP 0 411 121; EP 0 446 788; GB 1 226 540; JP 55037917; WO 81/00764; WO 86/06163; WO 93/03666; WO 98/01730; WO 98/08431 and WO 99/39166;

Lunar Baby Thermometer by Duck Young Kong available at http://www.duckyoungkong.com;

SWAN™ thermometer, commercially available from Medisim Ltd. of Neve Ilan, Israel;

Accu-Touch Forehead Thermometer, commercially available from Medisim Ltd. of Neve Ilan, Israel;

FHT™ Instant Thermometer, commercially available from Medisim Ltd. of Neve Ilan, Israel;

"Surface Temperature Scanner STS-100-F/C & 101-C," Omega Medical Corporation, pamphlet, 2 pages, circa 1987;

"Sensor Touch Temporal Artery Thermometer--The Noninvasive Thermometer You Can Trust," 5 page brochure, Exergen Corporation, 1999;

Wei, D., et al., "Optimal Design of a Thermistor Probe for Surface Measurement of Cerebral Blood Flow, Transactions on Biomedical Engineering," 37(12):1159-1172, 1990;

Database WPI, Section PQ, Week 9505, Derwent Publications Ltd., London, GB, May 15, 1994, Abstract;

FirstTemp Intelligent Medical Systems, Model 2000A, Operation Manual, 8 pages, no date;

ConforTemp All-Family Instant Underarm Thermometer, Quick Start Instructions, 6 pages, no date;

FirstTemp Clinical Thermometer, Technical Manual, 24 pages, no date;

Omega Medical Surface Temperature Scanner STS-100-F/C & 101-C, 2 pages, no date;

DET TRONICS, Optical Calibration, Oct. 1987.

Model 1M Thermopile Detector, Dexter Research Center, Technical Description, Oct. 1980;

Fraden, Jacob, "Application of Piezo/Pyroelectric Films in Medical Transducers," J. Clinical Engineering, 13 (3):133-138 (1988);

Houdas, Y. And Ring, E.F.J., "Human Body Temperature, Its Measurement and Regulation," Plenum Press, N.Y., p. 83, 1982

Looney, Jr., Joseph M. and Pompei, Francisco, "Ear Thermometry," Medical Electronics, Jun. 1989;

Surface Temperature Scanner, Models: STS100-F/C, STS-101-C, User Manual, 9 pages, no date;

Proceedings of the Eighth Annual Conference of the IEEE/Engineering in Medicine and Biology Society, vol. 3 of 3, Nov. 7-10, 1986;

Ryan, Marybeth, "The Equine Infrared Thermographic Scanner: Assuring Performance of the Equine Athlete," Exergen Corporation, 4 pages, 1989;

Pompei, Marybeth, "Temperature Assessment via the Temporal Artery: Validation of a New Method," Exergen Corporation, 1999;

Sketch of Radiation Detector Manufactured by IR-ONICS Corporation and distributed by Dermatherm Corporation, pre 1983;

Kuzucu, Etham Y., "Measurement of Temperature," Int. Anesthesiol Clin. 3(3): 435-449 (1965);

PET.TM., Physician's Electronic Thermometer, Phillips Bio-Medical, Inc., Specifications, 2 pgs., no date;

Pompei and M. Pompei, "Non-invasive temporal artery thermometry: Physics, physiology, and clinical accuracy," in Proceedings of SPIE, M. R. Dury, E. T. Theocharous, N. J. Harrison, M. Hilton, and N. Fox, Eds., vol. 5405, Apr. 2004, pp.61-67;

D. Fiala, K. J. Lomas, and M. Stohrer, "A computer model of human thermoregulation for a wide range of environmental conditions: the passive system." J Appl Physiol, vol. 87, No. 8750-7587, pp. 1957-1972, 1999;

Y. H. Chiok, E. Y.-K. Ng, and V. V. Kulish, "Global bioheat model for quick evaluation of the human physiological thermal profiles under differing conditions." J Med Eng Technol, vol. 26, No. 0309-1902, pp. 231-238, 2002;

Z.-S. Deng and J. Liu, "Mathematical modeling of temperature mapping over skin surface and its implementation in thermal disease diagnostics." Comput Biol Med, vol. 34, No. 0010-4825, pp. 495-521, 2004; and

S. B. Wilson and V. A. Spence, "A tissue heat transfer model for relating dynamic skin temperature changes to physiological parameters." Phys Med Biol, vol. 33, No. 0031-9155, pp. 895-912, 1988.


SUMMARY OF THE INVENTION

The present invention seeks to provide an easy to use thermometer for measurement of temperature of a person.

There is thus provided in accordance with a preferred embodiment of the present invention a thermometer useful for measuring the temperature of a patient, the thermometer including a housing, at least one temperature sensor assembly including a plurality of external patient skin contacting surfaces, and a mounting element for mounting the at least one temperature sensor assembly on the housing and being operative for mutually independently mounting each of the plurality of external patient skin contacting surfaces to be independently arrangeable in a spatial orientation which may be independently different from the spatial orientation of the external patient skin contacting surface of each other of the plurality of external patient skin contacting surfaces.

In accordance with a preferred embodiment of the present invention, each of the at least one temperature sensor assembly includes a plurality of temperature detectors, each temperature detector being associated with one of the plurality of external patient skin contacting surfaces. Additionally, the plurality of external patient skin contacting surfaces are independently arrangeable in response to at least one of contact with a patient skin surface and bending of a user appendage on which the housing is mounted.

Preferably, at least one of the housing and the mounting element provides thermal insulation between individual ones of the external patient skin contacting surfaces. Additionally, at least one of the housing and the mounting element is adapted to provide thermal insulation between the temperature sensory assembly and a user's appendage.

Preferably, the housing is ambidextrous. Additionally, the housing includes a finger cot. Additionally, the thermometer also includes a user interface and temperature display portion. Additionally, the user interface and temperature display portion also includes an LCD display. Preferably, the user interface and temperature display portion also includes an on-off push button. Preferably, the thermometer also includes a buzzer. Preferably, the thermometer also includes a battery. Preferably, the thermometer also includes a printed circuit board.

There is also provided in accordance with another preferred embodiment of the present invention a thermometer useful for measuring the temperature of a patient, the thermometer including a user appendage mountable housing, at least one temperature sensor assembly including a plurality of external patient skin contacting surfaces, and a mounting element for mounting the plurality of external patient skin contacting surfaces, the mounting element being flexible in response to flexing of an appendage onto which the housing is mounted,

Preferably, the user appendage mountable housing is mountable on the finger of a user. Additionally, the user appendage mountable housing is adapted to accommodate various different finger sizes.

In accordance with a preferred embodiment of the present invention, the mounting element is adapted to adopt a curvature which is responsive to a curvature of a patient body surface engaged thereby and to the flexing of a user appendage onto which the housing is mounted. Preferably, the user appendage mountable housing is stretchable. Additionally, the user appendage mountable housing is of a width that is not significantly greater than the width of an average user's finger.

Preferably, the mounting element is operative for mutually independently mounting each of the plurality of external patient skin contacting surfaces to be independently arrangeable in a spatial orientation which may be independently different from the spatial orientation of the external patient skin contacting surface of each other of the plurality of external patient skin contacting surfaces. Additionally, each of the at least one temperature sensor assembly includes a plurality of temperature detectors, each temperature detector being associated with one of the plurality of external patient skin contacting surfaces. Additionally, the plurality of external patient skin contacting surfaces are independently arrangeable in response to at least one of contact with a patient skin surface and bending of a user appendage on which the housing is mounted.

Preferably, at least one of the housing and the mounting element provides thermal insulation between individual ones of the external patient skin contacting surfaces. Additionally, at least one of the housing and the mounting element is adapted to provide thermal insulation between the temperature sensory assembly and a user's appendage.

Preferably, the housing is ambidextrous. Additionally, the housing includes a finger cot. Additionally, the thermometer also includes a user interface and temperature display portion. Additionally, the user interface and temperature display portion also includes an LCD display. Preferably, the user interface and temperature display portion also includes an on-off push button. Preferably, the thermometer also includes a buzzer. Additionally, the thermometer also includes a battery. Preferably, the thermometer also includes a printed circuit board.

There is further provided in accordance with yet another preferred embodiment of the present invention a thermometer useful for measuring the temperature of a patient, the thermometer including a user appendage mountable housing including a patient body surface engagement and temperature sensing portion arranged to be facing in a first direction away from the user appendage and a user interface and temperature display portion arranged to be facing away from the user appendage in a second direction, generally opposite to the first direction, and an at least partially closed ring portion supporting the body surface engagement and temperature sensing portion and the user interface and temperature display portion for mounting onto an appendage of a user.

In accordance with a preferred embodiment of the present invention, the user appendage mountable housing is mountable on the finger of a user. Preferably, the user appendage mountable housing is adapted to accommodate various different finger sizes. Additionally, the mounting element is adapted to adopt a curvature which is responsive to a curvature of a patient body surface engaged thereby and to the flexing of a user appendage onto which the housing is mounted. Preferably, the user appendage mountable housing is stretchable. Preferably, the user appendage mountable housing is of a width that is not significantly greater than the width of an average user's finger.

In accordance with a preferred embodiment of the present invention, the mounting element is operative for mutually independently mounting each of the plurality of external patient skin contacting surfaces to be independently arrangeable in a spatial orientation which may be independently different from the spatial orientation of the external patient skin contacting surface of each other of the plurality of external patient skin contacting surfaces. Preferably, each of the at least one temperature sensor assembly includes a plurality of temperature detectors, each temperature detector being associated with one of the plurality of external patient skin contacting surfaces. Additionally, the plurality of external patient skin contacting surfaces are independently arrangeable in response to at least one of contact with a patient skin surface and bending of a user appendage on which the housing is mounted.

Preferably, at least one of the housing and the mounting element provides thermal insulation between individual ones of the external patient skin contacting surfaces. Additionally, at least one of the housing and the mounting element is adapted to provide thermal insulation between the temperature sensory assembly and a user's appendage. Preferably, the housing is ambidextrous. Preferably, the housing includes a finger cot. Additionally, the thermometer also includes a user interface and temperature display portion. Additionally, the user interface and temperature display portion also includes an LCD display. Preferably, the user interface and temperature display portion also includes an on-off push button. Preferably, the thermometer also includes a buzzer. Additionally, the thermometer also includes a battery. Additionally, the thermometer also includes a printed circuit board.

There is yet further provided in accordance with still another preferred embodiment of the present invention a thermometer useful for measuring the temperature of a patient, the thermometer including a user appendage mountable housing, at least one temperature sensor assembly including a plurality of external patient skin contacting surfaces, and a mounting element for mounting the at least one temperature sensor assembly on the housing and being operative for mutually independently mounting each of the plurality of external patient skin contacting surfaces responsively to the configuration of both the patient skin contacting surface and the user's appendage.

In accordance with a preferred embodiment of the present invention, the user appendage mountable housing is mountable on the finger of a user. Preferably, the user appendage mountable housing is adapted to accommodate various different finger sizes. Preferably, the mounting element is adapted to adopt a curvature which is responsive to a curvature of a patient body surface engaged thereby and to the flexing of a user appendage onto which the housing is mounted. Preferably, the user appendage mountable housing is stretchable. Preferably, the user appendage mountable housing is of a width that is not significantly greater than the width of an average user's finger.

In accordance with a preferred embodiment of the present invention, the mounting element is operative for mutually independently mounting each of the plurality of external patient skin contacting surfaces to be independently arrangeable in a spatial orientation which may be independently different from the spatial orientation of the external patient skin contacting surface of each other of the plurality of external patient skin contacting surfaces. Preferably, each of the at least one temperature sensor assembly includes a plurality of temperature detectors, each temperature detector being associated with one of the plurality of external patient skin contacting surfaces.

Preferably, the plurality of external patient skin contacting surfaces are independently arrangeable in response to at least one of contact with a patient skin surface and bending of a user appendage on which the housing is mounted. Additionally, at least one of the housing and the mounting element provides thermal insulation between individual ones of the external patient skin contacting surfaces. Preferably, at least one of the housing and the mounting element is adapted to provide thermal insulation between the temperature sensory assembly and a user's appendage. Preferably, the housing is ambidextrous. Preferably, the housing includes a finger cot.

There is also provided in accordance with another preferred embodiment of the present invention a thermometer useful for measuring the temperature of a patient, the thermometer including a user appendage mountable housing, at least one temperature sensor assembly including at least one external patient skin contacting surface thermally connected to at least one temperature detector in a manner that the temperature sensed at the at least one temperature detector generally represents an average of temperatures at the at least one external patient skin contacting surface, and a mounting element for mounting the at least one temperature sensor assembly on the housing.

In accordance with a preferred embodiment of the present invention, the user appendage mountable housing is mountable on the finger of a user. Additionally, the user appendage mountable housing is adapted to accommodate various different finger sizes. Preferably, the mounting element is adapted to adopt a curvature which is responsive to a curvature of a patient body surface engaged thereby and to the flexing of a user appendage onto which the housing is mounted.

Preferably, the user appendage mountable housing is stretchable. Additionally, the user appendage mountable housing is of a width that is not significantly greater than the width of an average user's finger.

In accordance with a preferred embodiment of the present invention, the mounting element is operative for mutually independently mounting each of the at least one external patient skin contacting surface to be independently arrangeable in a spatial orientation which may be independently different from the spatial orientation of the external patient skin contacting surface of each other of the at least one external patient skin contacting surface. Preferably, each of the at least one temperature sensor assembly includes a plurality of temperature detectors, each temperature detector being associated with one of the at least one external patient skin contacting surface.

Preferably, the at least one external patient skin contacting surface are independently arrangeable in response to at least one of contact with a patient skin surface and bending of a user appendage on which the housing is mounted. Additionally, at least one of the housing and the mounting element provides thermal insulation between individual ones of the at least one external patient skin contacting surface. Additionally, at least one of the housing and the mounting element is adapted to provide thermal insulation between the temperature sensory assembly and a user's appendage.

Preferably, the housing is ambidextrous. Preferably, the housing includes a finger cot. Additionally, the thermometer also includes a user interface and temperature display portion. Preferably, the user interface and temperature display portion also includes an LCD display. Preferably, the user interface and temperature display portion also includes an on-off push button. Preferably, the thermometer also includes a buzzer.

Additionally, the thermometer also includes a battery. Additionally, the thermometer also includes a printed circuit board.

BRIEF DESCRIPTION OF THE DRAWINGS

The present invention will be understood and appreciated more fully from the following detailed description, taken in conjunction with the drawings in which:

Fig. 1 is a simplified illustration of the use of a thermometer constructed and operative in accordance with a preferred embodiment of the present invention for measuring temperature of a patient by engagement with a generally convex body surface such as the forehead of a patient;

Fig. 2 is a simplified illustration of the use of a thermometer constructed and operative in accordance with a preferred embodiment of the present invention for measuring temperature of a patient by engagement with a generally concave body surface such as the armpit of a patient;

Figs. 3A & 3B are simplified illustrations showing engagement of the thermometer of Fig. 1 with a finger of a person measuring temperature;

Figs. 4A and 4B are simplified exploded pictorial illustrations of the thermometer of Figs. 1-3B, taken in respective downward and upward facing directions;

Figs. 5A and 5B are simplified pictorial illustrations of an interior structural element, forming part of the thermometer of Figs. 1 - 4B, taken in respective downward and upward facing directions;

Fig. 5C is a simplified pictorial illustration of the interior structural element of Figs. 5A and 5B, taken in a generally opposite direction;

Fig. 5D is a simplified sectional illustration of the interior structural element of Figs. 5A, 5B and 5C, taken along lines D - D in Figs. 5A, 5B and 5C;

Fig. 5E, is a simplified sectional illustration of the interior structural element of Figs. 5A, 5B and 5C, taken along lines E - E in Figs. 5A, 5B and 5C;

Figs. 6A and 6B are pictorial illustrations of a sensor assembly, forming part of the thermometer of Figs. 1 - 4B, taken along different directions;

Fig. 6C is a sectional illustration of the sensor assembly of Figs. 6A and 6B, taken along lines C - C in Fig. 6A.

Figs. 7A and 7B are simplified pictorial illustrations of an exterior structural element, forming part of the thermometer of Figs. 1 - 4B, taken in respective downward and upward facing directions;

Fig. 7C is a simplified pictorial illustration corresponding to Fig. 7A but taken in a generally opposite direction;

Fig. 7D is a simplified sectional illustration of the exterior structural element of Figs 7A, 7B and 7C, taken along lines D - D in Figs. 7A, 7B and 7C;

Fig. 7E, is a simplified sectional illustration of the exterior structural element of Figs 7A, 7B and 7C, taken along lines E - E in Figs. 7A, 7B and 7C;

Figs. 8A and 8B are simplified pictorial illustrations of a finger engagement ring, forming part of the thermometer of Figs. 1 - 4B, taken in respective downward and upward facing directions;

Fig. 8C is a simplified pictorial illustration corresponding to Fig. 8A but taken in a generally opposite direction;

Fig. 8D is a simplified sectional illustration of the finger engagement ring of Figs. 8A, 8B and 8C, taken along lines D - D in Fig. 8A;

Fig. 8E is a simplified sectional illustration of the finger engagement ring of Figs. 8A, 8B and 8C, taken along lines E - E in Fig. 8A;

Fig. 9A is a simplified pictorial illustration of the thermometer of Figs. 1-8E;

Fig. 9B is a simplified side view illustration of the thermometer of Fig. 9A taken along arrow B in Fig 9A;

Fig. 9C is a sectional illustration of the thermometer of Figs. 9A & 9B, taken along lines C - C in Fig. 9A;

Fig. 10A is a simplified pictorial illustration of a thermometer constructed and operative in accordance with another preferred embodiment of the present invention for measuring temperature of a patient;

Fig. 10B is a simplified side view illustration of the thermometer of Fig. 10A taken along arrow B in Fig. 10A;

Fig. 10C is a sectional illustration of the thermometer of Figs. 10A & 10B, taken along lines C - C in Fig. 10A;

Fig. 11A is a simplified pictorial illustration of a thermometer constructed and operative in accordance with yet another preferred embodiment of the present invention for measuring temperature of a patient;

Fig. 11B is a simplified side view illustration of the thermometer of Fig. 11A taken along arrow B in Fig. 11A;

Fig. 11C is a simplified sectional illustration of the thermometer of Figs. 11A & 11B, taken along lines C - C in Fig. 11A;

Figs. 12A and 12B are simplified exploded pictorial illustrations of a thermometer constructed and operative in accordance with still another preferred embodiment of the present invention for measuring temperature of a patient, taken respectively from the top and from the bottom;

Fig. 13A and 13B are simplified pictorial illustrations of an interior structural element of the thermometer of Figs. 12A and 12B taken in respective downward and upward facing directions;

Fig. 13C is a simplified pictorial illustration corresponding to Fig. 13A but taken in a generally opposite direction;

Fig. 13D is a simplified sectional illustration of the thermometer of Figs. 13A, 13B and 13C, taken along lines D - D in Fig. 13A;

Fig. 13E is a simplified sectional illustration of the thermometer of Figs. 13A, 13B and 13C, taken along lines E - E in Figs. 13A;

Figs. 14A and 14B are pictorial illustrations of a sensor assembly, taken along different directions;

Fig. 14C is a sectional illustration of the sensor assembly of Figs. 14A and 14B, taken along lines C - C in Fig. 14A;

Fig. 14D is a sectional illustration of the sensor assembly of Figs. 14A and 14B, taken along lines D - D in Fig. 14B;

Figs. 15A and 15B are simplified pictorial illustrations of an exterior structural element taken in respective downward and upward facing directions;

Fig. 15C is a simplified pictorial illustration corresponding to Fig. 15A but taken in a generally opposite direction;

Fig. 15D is a simplified sectional illustration of the exterior structural element of Figs 15A, 15B and 15C, taken along lines D - D in Fig. 15A;

Fig. 15E is a simplified sectional illustration of the exterior structural element of Figs 15A, 15B and 15C, taken along lines E - E in Fig. 15A;

Figs. 16A and 16B are simplified pictorial illustrations of a finger engagement ring element, taken in respective downward and upward facing directions;

Fig. 16C is a simplified pictorial illustration corresponding to Fig. 16A but taken in a generally opposite direction;

Fig. 16D is a simplified sectional illustration of the finger engagement ring element of Figs 16A, 16B and 16C, taken along lines D - D in Fig. 16A;

Fig. 16E is a simplified sectional illustration of the finger engagement ring element of Figs 16A, 16B and 15C, taken along lines E - E in Fig. 15A;

Figs. 17A and 17B are simplified exploded pictorial illustrations of yet another embodiment of a thermometer constructed and operative in accordance with a preferred embodiment of the present invention for measuring temperature of a patient, taken respectively from the top and from the bottom;

Figs. 18A and 18B are pictorial illustrations of a sensor assembly, forming part of the thermometer of Figs. 17A and 17B;

Fig. 18C is a sectional illustration of the sensor assembly of Figs. 18A and 18B, taken along lines C - C in Fig. 18B;

Fig. 18D is a sectional illustration of the sensor assembly of Figs. 18A and 18B, taken along lines D - D in Fig. 18B;

Fig. 19 is a simplified pictorial illustration of a thermometer constructed and operative in accordance with another preferred embodiment of the present invention for measuring temperature of a patient;

Figs. 20A and 20B are simplified side view illustrations taken along directions indicated by arrows A and B in Fig. 19;

Figs. 20C and 20D simplified sectional illustrations taken along respective lines C - C and D - D in Fig. 19; and

Figs. 21A, 21B and 21C are simplified pictorial illustrations showing the thermometer of Fig. 19 engaged with a finger of a person measuring temperature as a finger cot is slipped onto the finger.

DETAILED DESCRIPTION OF PREFERRED EMBODIMENTS

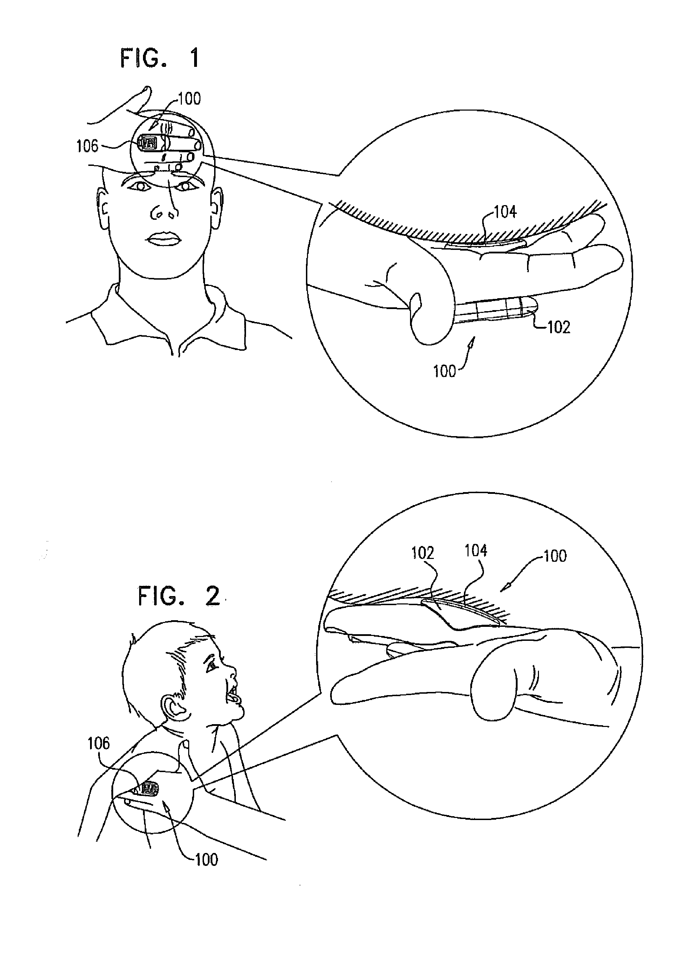

Reference is now made to Figs. 1, 2, 3A & 3B, which are simplified illustrations of the use of a thermometer constructed and operative in accordance with a preferred embodiment of the present invention for measuring temperature of a patient. As seen in Figs. 1 - 3B, there is provided an at least partially finger surrounding thermometer 100 including a housing 102 having a body surface curvature responsive engagement and temperature sensing portion 104 arranged to be located facing opposite one finger portion and a user interface and temperature display portion 106 arranged to be located facing opposite another finger portion which located generally on the other side of the finger from the engagement portion 104.

As seen particularly in Figs. 1 and 2, it is a particular feature of the present invention that the body surface curvature responsive engagement and temperature sensing portion 104 is sufficiently flexible and resilient so as to be adapted for temperature sensing touching engagement with a generally convex body portion, such as a forehead, as shown in Fig. 1 and with a generally concave body portion, such as an armpit, as shown in Fig. 2.

It is a further particular feature of the present invention that in addition to the overall resiliency and flexibility of the body surface curvature responsive engagement and temperature sensing portion 104, individual temperature sensor assemblies 108 are mutually independently mounted in body surface curvature responsive engagement and temperature sensing portion 104, so as each to be independently arrangeable in a spatial orientation which may be independently different from the spatial orientation of each other of said plurality of temperature sensor assemblies.

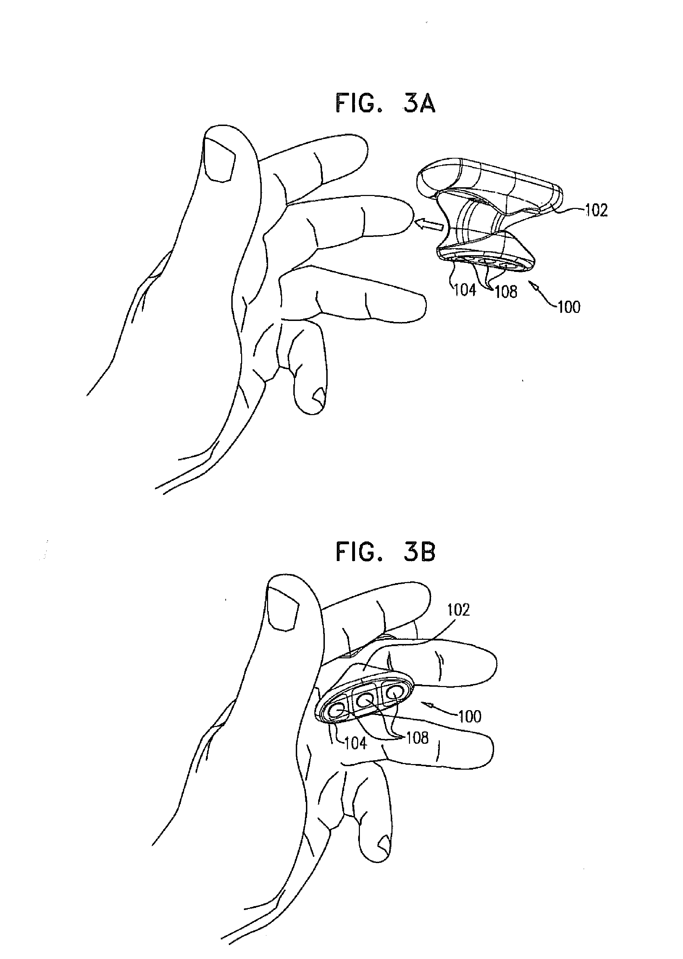

Figs 3A and 3B show that the thermometer 100 may be engaged with a finger of a person measuring temperature as a ring is slipped onto the finger. Preferably, the thermometer 100 sits on the proximal phalange of the finger as shown. It is preferred that the sensor assemblies 108 be arranged with respect to the remainder of the thermometer 100 in a symmetric arrangement such that the thermometer is ambidextrous.

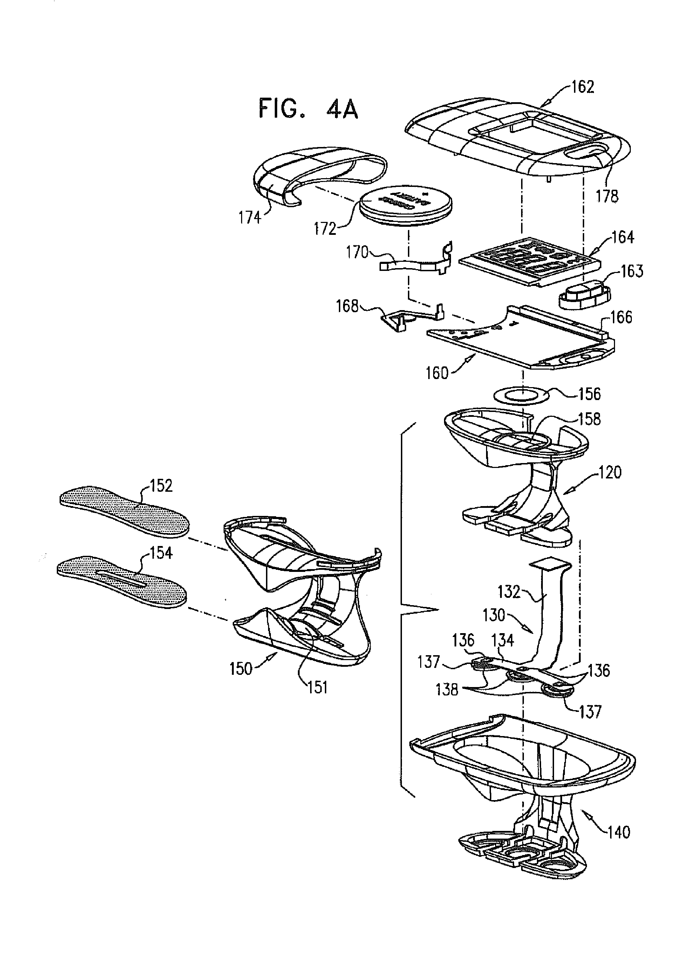

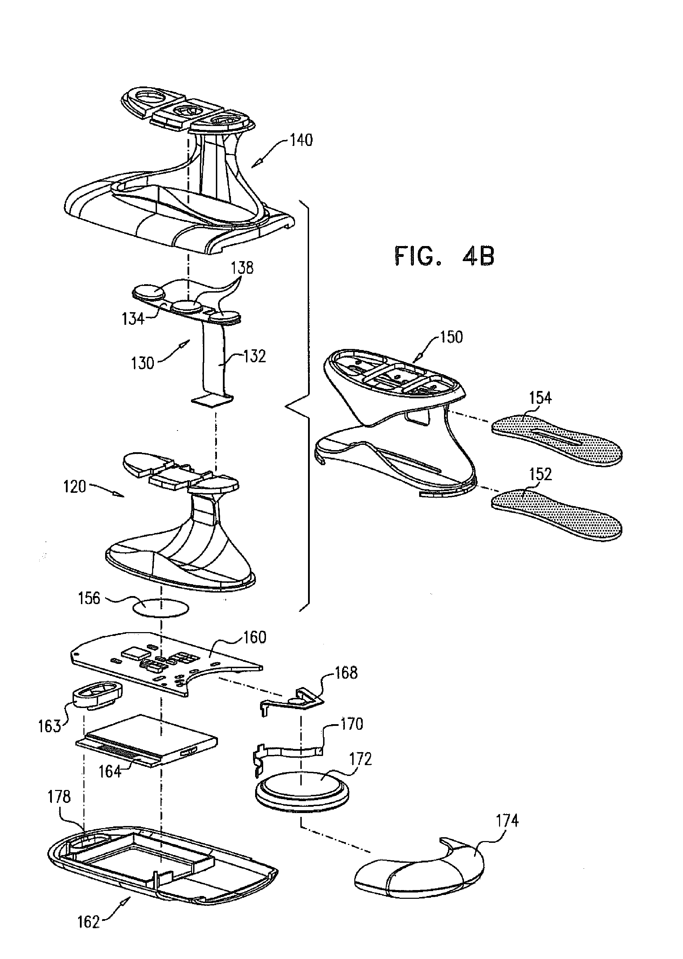

Reference is now made to Figs. 4A and 4B, which are simplified, exploded pictorial illustrations of an embodiment of the thermometer of Figs. 1-3B, taken respectively from the top and from the bottom. As seen in Figs. 4A & 4B, the thermometer 100 comprises an interior structural element 120, typically integrally injection molded of, for example, TECHNYL® A 205F commercially available from Rhodia Engineering Plastics SA of Paris, France. A sensor assembly 130 is mounted rearwardly of and below interior structural element 120, in the sense of Fig. 4A. Sensor assembly 130 includes a flexible circuit connection portion 132 and a flexible sensor mounting portion 134.

A plurality of top facing sensors 136, preferably three in number, are mounted on a top surface of sensor mounting portion 134, and a corresponding plurality of bottom facing sensors 137 are mounted on an underneath surface of sensor mounting portion 134, generally opposite sensors 136. Sensors 136 and 137 are electrically connected to sensor mounting portion 134. A plurality of skin contact plates 138 corresponding to sensors 137 are preferably thermally adhesively mounted onto sensors 137. In alternative embodiments, top facing sensors 136 are obviated.

Interior structural element 120 and sensor assembly 130 are together located within an exterior structural element 140 and are lightly snap fitted together. A finger engagement ring element 150, preferably made of TPU such as the EMPILON® HA SERIES commercially available from EMPILON® of Taiwan, is overmolded over the assembled interior structural element 120, sensor assembly 130, and exterior structural element 140. The overmolding locks elements 120, 130, 140 and 150 together.

A plurality of generally radially inwardly directly flexible resilient ribs 151 may be integrally formed as part of finger engagement ring element 150 for low thermal conductivity finger engagement with fingers of various sizes. Resilient finger engagement pads 152 and 154 are optionally provided for adapting the fit of finger engagement ring element 150 to various finger sizes. Pads 152 and 154 preferably are formed of a good thermal insulator such as closed cell foam so as to provide good thermal insulation between a user's finger and sensors.

A buzzer 156 is preferably seated in a buzzer socket 158 formed on interior structural element 120.

A printed circuit board 160 is mounted onto a top cover panel 162 over exterior structural element 140. Various electrical components are mounted onto printed circuit board 160 including, inter alia, an on-off push button 163, an LCD display 164, an LCD connector 166 and battery mounting brackets 168 and 170. A battery 172 is mounted on brackets 168 and 170 and is accessed via a slidably removable battery cover 174. Top cover panel 162 is mounted onto exterior structural element 140 to enclose printed circuit board 160, LCD display 164, LCD connector 166 as well as bracket 170 and part of battery 172. Top cover panel 162 is preferably formed with an aperture 178 for accommodating push button 163, which protrudes therethrough.

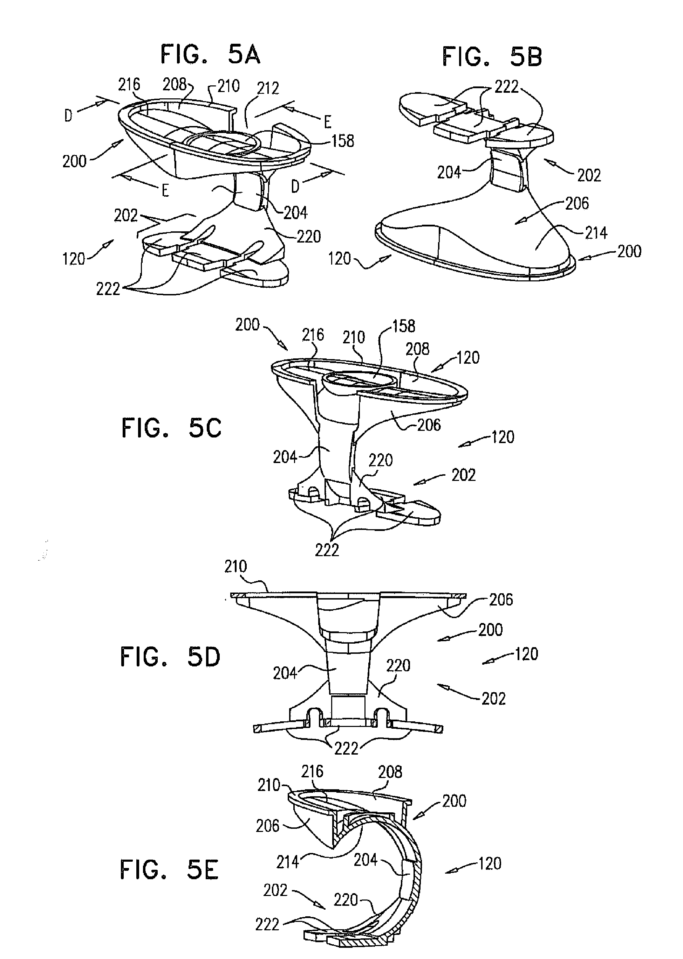

Reference is now made to Figs. 5A and 5B which are simplified pictorial illustrations of interior structural element 120 taken in respective downward and upward facing directions, Fig. 5C which is a simplified pictorial illustration corresponding to Fig. 5A but taken in a generally opposite direction, Fig. 5D, which is a simplified sectional illustration, taken along lines D - D in Figs. 5A, 5B and 5C and Fig. 5E, which is a simplified sectional illustration, taken along lines E - E in Figs. 5A, 5B and 5C.

As seen in Figs. 5A - 5E, interior structural element 120 preferably includes a top portion 200 and a bottom portion 202. Top portion 200 and bottom portion 202 are preferably integrally joined by a side portion 204.

Top portion 200 preferably includes a nearly-circumscribed wall portion 206, which is nearly surrounded by a nearly-circumferential wall 208, having a nearly-circumferential rim 210 at a top facing edge thereof. Wall 208 and rim 210 are interrupted by a gap 212. A downward facing surface 214 of wall portion 206 is configured generally as part of a cylindrical surface. An opposite, upward facing surface 216 of wall portion 206 is formed with a generally circular upstanding ring configuration which defines buzzer socket 158 (Figs. 4A & 4B).

Wall portion 206 is integrally formed with side portion 204 at an upper region of side portion 204. Side portion 204 is integrally formed at a lower region thereof with bottom portion 202. Bottom portion 202 preferably includes a flexible and resilient support portion 220 which is trifurcated to provide relatively independently flexible and resilient support to three sensor supports 222.

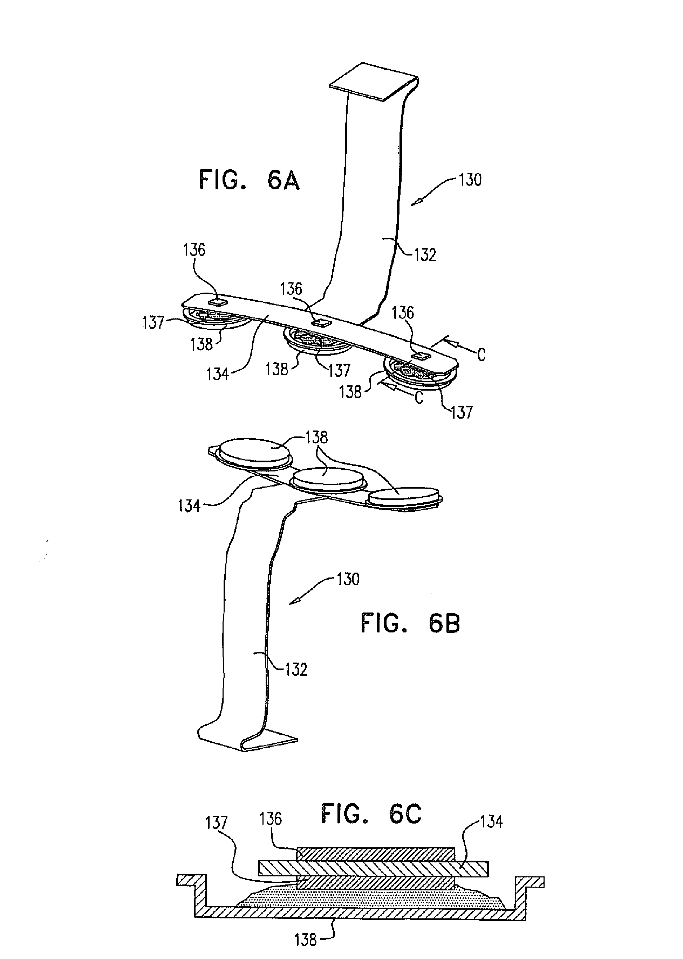

Reference is now made to Figs. 6A and 6B which are pictorial illustrations of sensor assembly 130, taken along different directions and to Fig. 6C which is a sectional illustration of sensor assembly 130, taken along lines C - C in Fig. 6A. As seen in Figs. 6A - 6C, the sensor assembly 130 includes a flexible circuit connection portion 132 and a flexible sensor mounting portion 134 extending generally perpendicular to portion 132 and integrally formed therewith.

A plurality of top facing sensors 136, preferably three in number are preferably SMT mounted on a top surface of portion 134, and a corresponding plurality of bottom facing sensors 137 are preferably SMT mounted on an underneath surface of sensor mounting portion 134, generally opposite sensors 136. As seen particularly clearly in Fig. 6C, skin contact plates 138 are preferably adhesively attached to sensors 137. Skin contact plates 138 are preferably formed of a heat conductive material such as metal, such as stainless steel 304 and are joined to sensors 137 by a highly thermally conductive adhesive such as Robnor Glue #PX439XS, commercially available from Robnor Resins Ltd. of Swindon, United Kingdom.

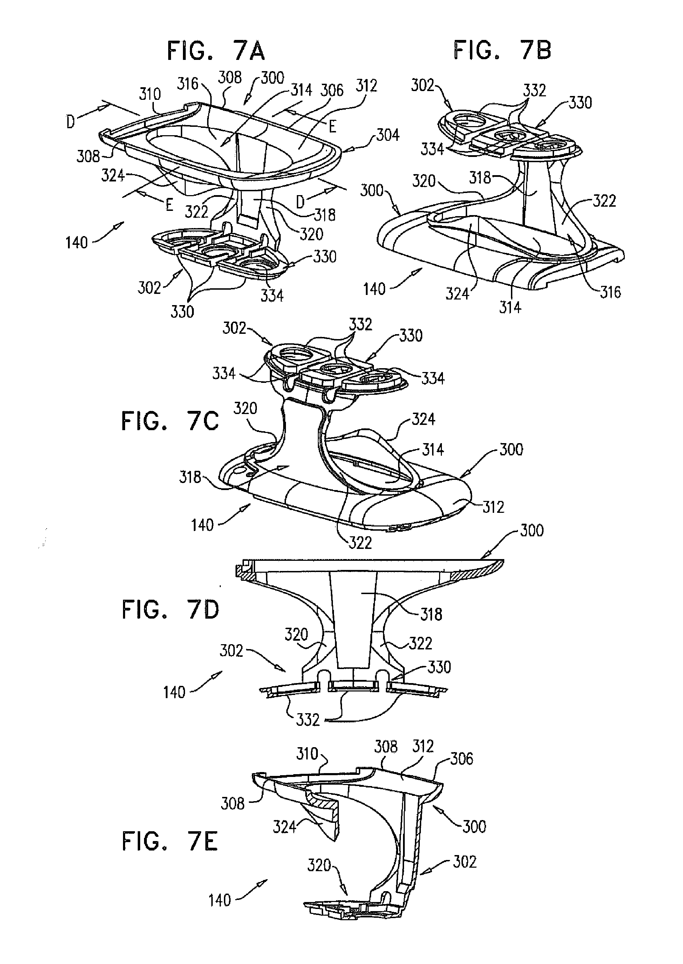

Reference is now made to Figs. 7A and 7B, which are simplified pictorial illustrations of exterior structural element 140 taken in respective downward and upward facing directions, to Fig. 7C which is a simplified pictorial illustration corresponding to Fig. 7A but taken in a generally opposite direction, to Fig. 7D, which is a simplified sectional illustration, taken along lines D - D in Figs. 7A, 7B and 7C, and to Fig. 7E, which is a simplified sectional illustration, taken along lines E - E in Figs. 7A, 7B and 7C.

As seen in Figs. 7A - 7E, exterior structural element 140 preferably includes a top portion 300 and a bottom portion 302. Top portion 300 and bottom portion 302 are preferably integrally joined.

Top portion 300 preferably includes a circumferential rim 304 which includes a main rim portion 306, two secondary side rim portions 308, which are slightly recessed with respect to the main rim portion 306 and an end rim portion 310, which is recessed with respect to side rim portions 308. A circumferential wall 312 extends between rim portions 306, 308 and 310 and about a central, generally oval aperture 314, which is, in turn, surrounded by a circumferential wall 316 extending downwardly from circumferential wall 312 in the sense of Fig. 7A.

Circumferential wall 316 includes a tapered and necked back portion 318 which is joined to bottom portion 302, side portions 320 and 322 on either side of back portion 318 and a forward lipped portion 324, seen particularly clearly in Fig. 7B.

Bottom portion 302 preferably includes a flexible and resilient support portion 330 which is trifurcated to provide relatively independently flexible and resilient support to three skin contact plate sockets 332, each of which includes a plate accommodating aperture 334.

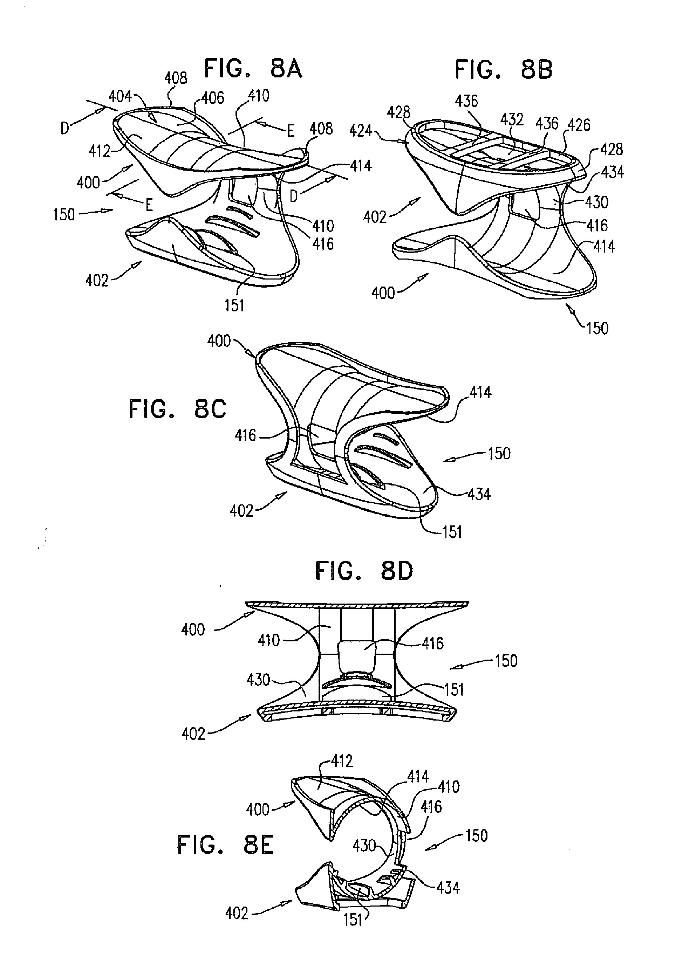

Reference is now made to Figs. 8A and 8B which are simplified pictorial illustrations of finger engagement ring element 150, which is overmolded over the assembled interior structural element 120, sensor assembly 130 and exterior structural element 140, taken in respective downward and upward facing directions, to Fig. 8C which is a simplified pictorial illustration corresponding to Fig. 8A but taken in a generally opposite direction, to Fig. 8D, which is a simplified sectional illustration, taken along lines D - D in Figs. 8A, 8B and 8C, and to Fig. 8E, which is a simplified sectional illustration, taken along lines E - E in Figs. 8A, 8B and 8C.

As seen in Figs. 8A - 8E, finger engagement ring element 150 preferably includes a top portion 400 and a bottom portion 402. Top portion 400 and bottom portion 402 are preferably integrally joined.

Top portion 400 preferably includes a circumferential wall 404 which includes a lipped rim portion 406, which is seen clearly in Fig. 8A, two secondary side rim portions 408 and a side wall portion 410. Circumferential wall 404 surrounds an interior wall 412. A downward facing finger engaging surface 414 of wall 412 is configured generally as part of a cylindrical surface. A plurality of generally radially inwardly directly flexible resilient ribs 151 (Figs. 4A & 4B) may be integrally formed with surface 414.

Side wall portion 410 is preferably tapered and necked and is joined to bottom portion 402, a generally square cut out 416 being defined generally at this junction.

Bottom portion 402 preferably includes a circumferential wall 424 which includes a lipped portion 426, which is seen clearly in Fig. 8B, two secondary side portions 428 and a side wall portion 430. Circumferential wall 424 surrounds an interior portion 432. Interior portion 432 surrounds flexible sensor mounting portion 134, sensors 136 and 137, skin contact plates 138, sensor supports 222 and skin contact plate sockets 332.

Radially interiorly of flexible sensor mounting portion 134, sensors 136 and 137, skin contact plates 138, sensor supports 222 and skin contact plate sockets 332, interior portion 432 includes an upward facing finger engaging surface portion 434, which is configured generally as part of a cylindrical surface.

Interior portion 432 also includes a pair of flexible and resilient ribs 436 which are located radially exteriorly of flexible sensor mounting portion 134 and alongside and between separate independently resiliently and flexibly mounted individual sensor supports 222 and skin contact plate sockets 332 which in turn support sensors 137 and skin contact plates 138.

Side wall portion 430 is preferably tapered and necked and is joined to side wall portion 410 of top portion 400.

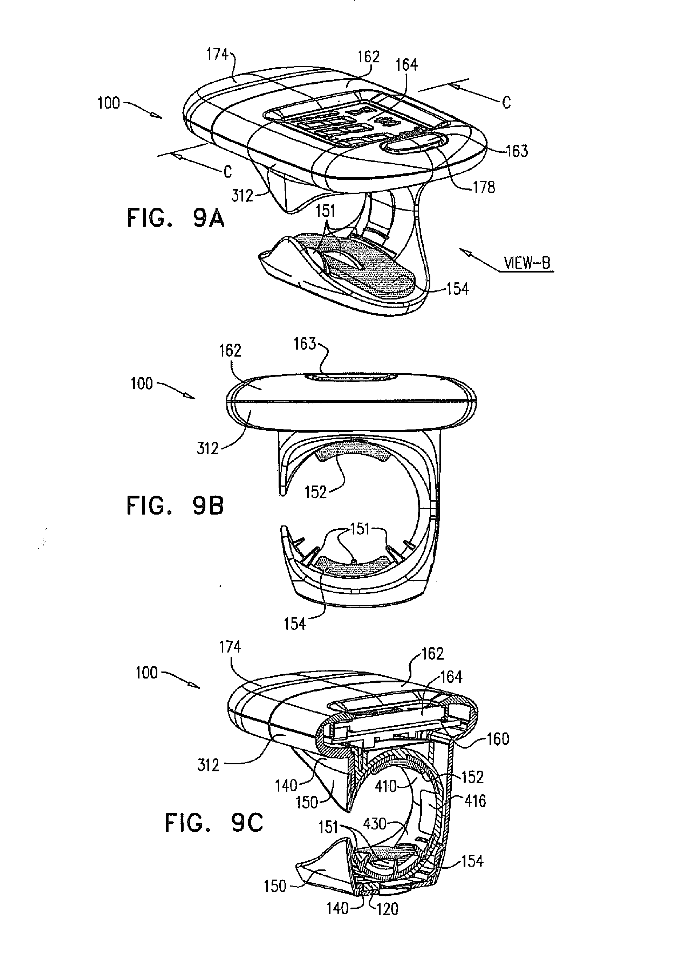

Figs. 9A, 9B and 9C illustrate the thermometer of Figs. 1-8E. Fig. 9A is a simplified pictorial illustration of the thermometer and Fig. 9B is a simplified side view illustration taken along arrow B in Fig 9A. Fig. 9C is a sectional illustration of the thermometer of Figs. 9A & 9B, taken along lines C - C in Fig. 9A.

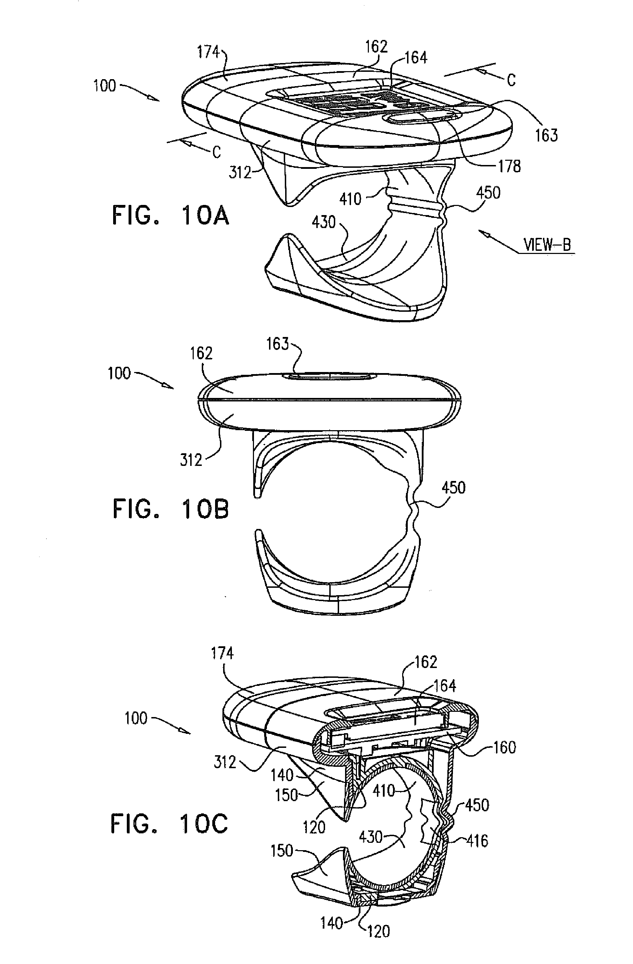

Reference is now made to Figs. 10A, 10B and 10C, which illustrate an alternative embodiment of the thermometer of Figs. 1-8E wherein there is provided a resiliently stretchable and bendable side ring portion 450 which replaces ribs 151 and pads 152 and 154 in the embodiment of Figs. 1-8E. Alternatively, the resiliently stretchable and bendable side ring portion 450 may be provided in addition to ribs 151 and pads 152 and 154 in the embodiment of Figs. 1-8E. Fig. 10A is a simplified pictorial illustration of the alternative embodiment of the thermometer of Figs. 10A, and Fig. 10B is a simplified side view illustration of the thermometer of Fig. 10A taken along arrow B in Fig. 10A. Fig. 10C is a sectional illustration of the thermometer of Figs. 10A & 10B, taken along lines C - C in Fig. 10A.

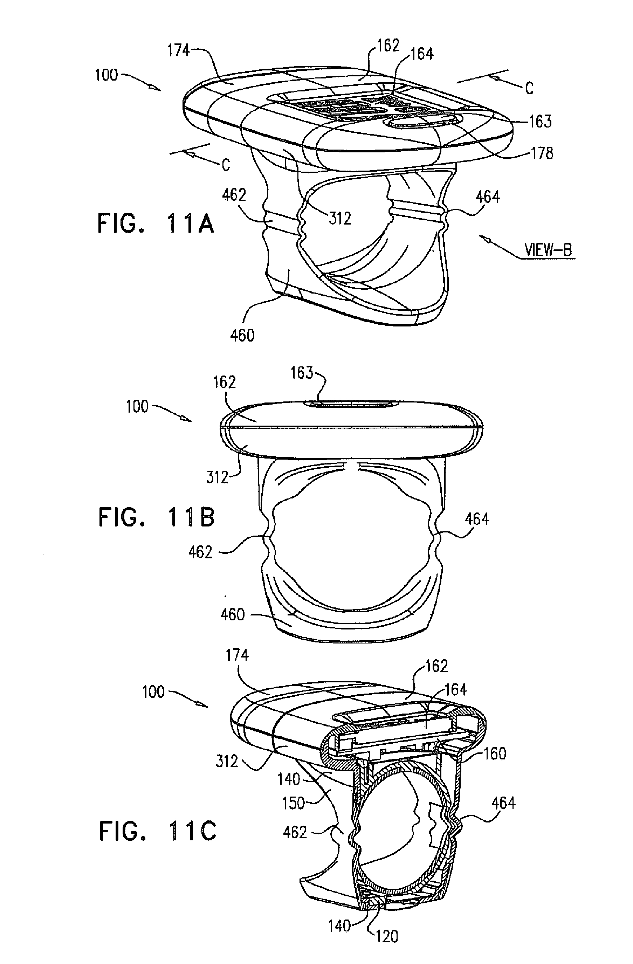

Reference is now made to Figs. 11A, 11B and 11C, which illustrate another alternative embodiment of the thermometer of Figs. 1-8E wherein a closed ring 460 is provided with a pair of resiliently stretchable and bendable side ring portions 462 and 464. Fig. 11A is a simplified pictorial illustration of the alternative embodiment of the thermometer of Figs. 1-8E and Fig. 11B is a simplified side view illustration of the thermometer of Fig. 11A taken along arrow B in Fig. 11AFig. 11C is a sectional illustration of the thermometer of Figs. 11A & 11B, taken along lines C - C in Fig. 11A.

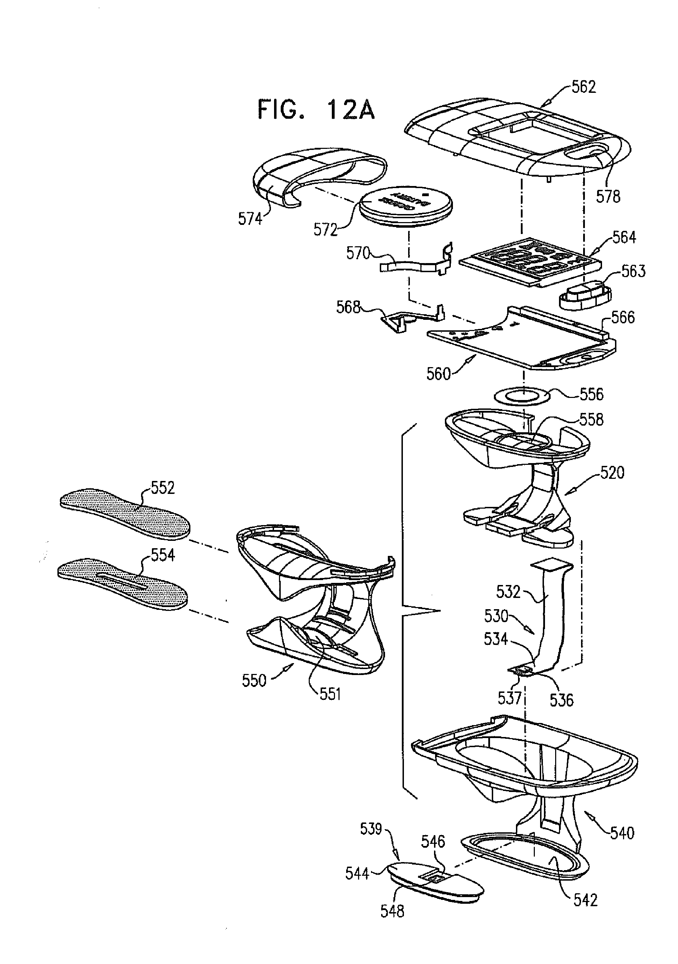

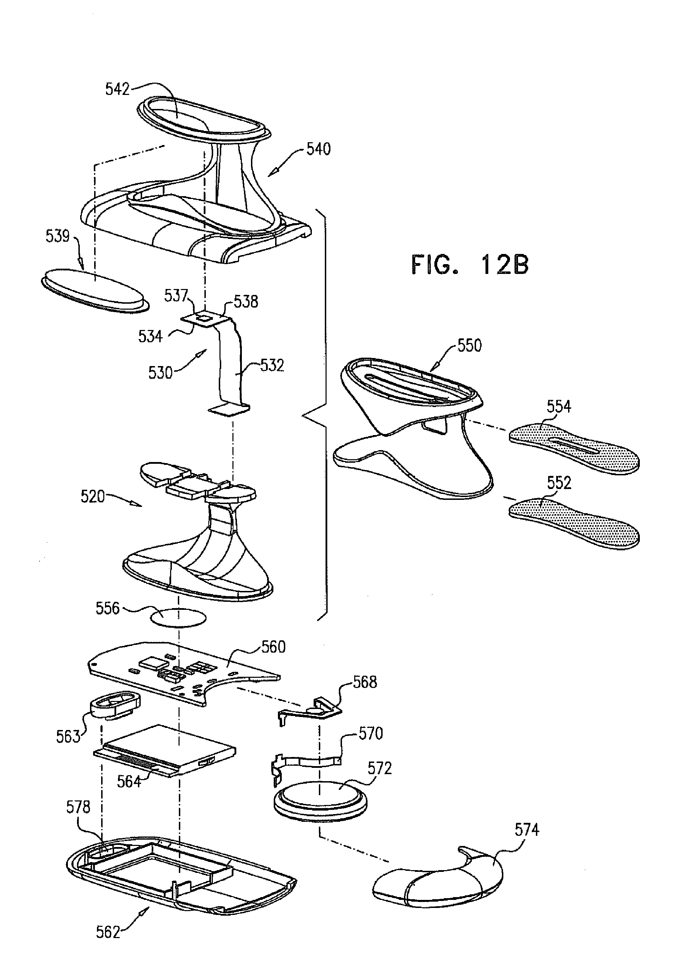

Reference is now made to Figs. 12A and 12B, which are simplified, exploded pictorial illustrations of another embodiment of the thermometer of Figs. 1 & 2, taken respectively from the top and from the bottom. As seen in Figs. 12A & 12B, the thermometer 500 comprises an interior structural element 520, typically integrally injection molded of, for example, TECHNYL® A 205F commercially available from Rhodia Engineering Plastics SA of Paris, France. A sensor assembly 530 is mounted rearwardly of and below interior structural element 520, in the sense of Fig. 12A. Sensor assembly 530 includes a flexible circuit connection portion 532 and a flexible sensor mounting portion 534.

A top facing sensor 536 is mounted on a top surface of portion 534, and a corresponding bottom facing sensor 537 is mounted on a bottom surface of portion 534, generally opposite sensor 536. Sensors 536 and 537 are electrically connected to sensor mounting portion 534. In alternative embodiments, top facing sensors 536 are obviated.

A temperature averaging skin surface contact pad 539, preferably made of a resilient, flexible, material, such as silicon rubber, is provided. Lateral heat conducting copper elements are embedded within surface contact pad 539 to provide anisotropic lateral thermal conductivity throughout the length of pad 539, which conductivity is significantly greater than the transverse thermal conductivity across the thickness of pad 539.

Interior structural element 520 and sensor assembly 530 are together located within an exterior structural element 540 and are lightly snap fitted together. Exterior structural element 540 is preferably formed with a peripheral socket 542 in which temperature averaging pad 539 is seated. As seen particularly in Fig. 12A, an inner facing surface 544 of pad 539 is preferably formed with a recess 546 for receiving sensor mounting portion 534. Disposed within recess 546 there is preferably provided a further recess 548 for accommodating bottom facing sensor 537 which is adhered thereto by a suitable thermally conductive adhesive such as Robnor Glue #PX439XS, commercially available from Robnor Resins Ltd. of Swindon, United Kingdom.

A finger engagement ring element 550, preferably made of TPU such as the EMPILON® HA SERIES commercially available from EMPILON® of Taiwan, is overmolded over the assembled interior structural element 520, sensor assembly 530, exterior structural element 540 and surrounding the periphery of temperature averaging skin surface contact pad 539. The overmolding locks at least elements 520, 530, 534, 537, 539, 540 and 550 together.

A plurality of generally radially inwardly directly flexible resilient ribs 551 may be integrally formed as part of finger engagement ring element 550 for low thermal conductivity finger engagement with fingers of various sizes. Resilient finger engagement pads 552 and 554 are optionally provided for adapting the fit of finger engagement ring element 550 to various finger sizes. Pads 552 and 554 preferably are formed of a good thermal insulator such as closed cell foam so as to provide good thermal insulation between a user's finger and sensors.

A buzzer 556 is preferably seated in a buzzer socket 558 formed on interior structural element 520.

A printed circuit board 560 is mounted onto a top cover panel 562 over exterior structural element 540. Various electrical components are mounted between printed circuit board 560 and top cover panel 562 including, inter alia, an on-off push button 563, an LCD display 564, an LCD connector 566 and battery mounting brackets 568 and 570. A battery 572 is mounted on brackets 568 and 570 and is accessed via a slidably removable battery cover 574. Top cover panel 562 is mounted onto exterior structural element 540 to enclose printed circuit board 560, LCD display 564, LCD connector 566 as well as bracket 570 and part of battery 572. Top cover panel 562 is preferably formed with an aperture 578 for accommodating push button 563, which protrudes therethrough.

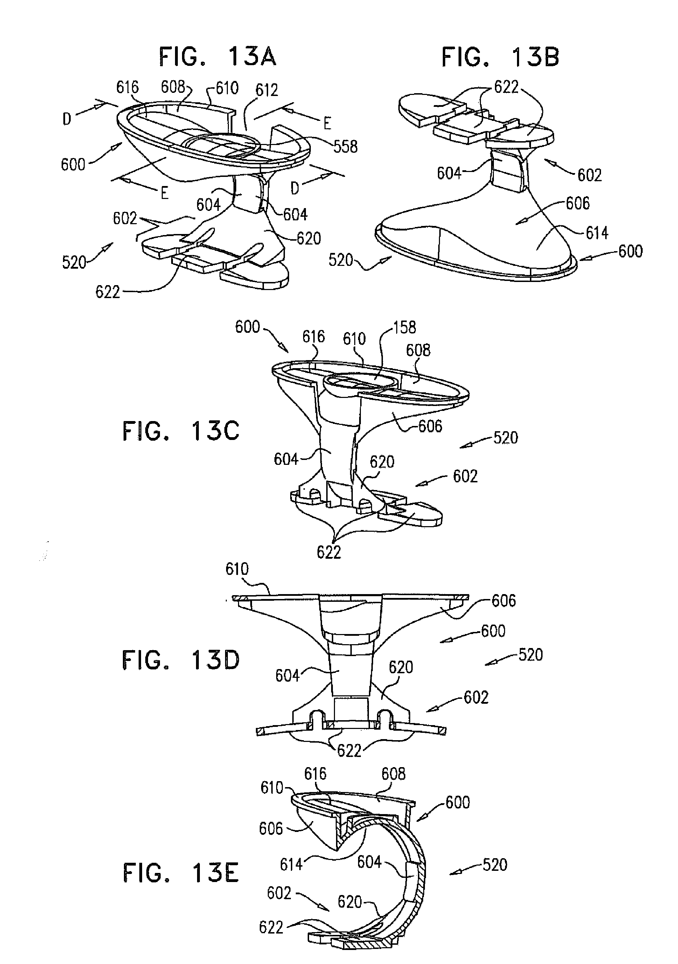

Reference is now made to Figs. 13A and 13B, which are simplified pictorial illustrations of interior structural element 520 taken in respective downward and upward facing directions, to Fig. 13C which is a simplified pictorial illustration corresponding to Fig. 13A but taken in a generally opposite direction, to Fig. 13D, which is a simplified sectional illustration, taken along lines D - D in Figs. 13A, 13B and 13C, and to Fig. 13E, which is a simplified sectional illustration, taken along lines E - E in Figs. 13A, 13B and 13C.

As seen in Figs. 13A - 13E, interior structural element 520 preferably includes a top portion 600 and a bottom portion 602. Top portion 600 and bottom portion 602 are preferably integrally joined by a side portion 604.

Top portion 600 preferably includes a nearly-circumscribed wall portion 606, which is nearly surrounded by a nearly-circumferential wall 608, having a nearly-circumferential rim 610 at a top facing edge thereof. Wall 608 and rim 610 are interrupted by a gap 612. A downward facing surface 614 of wall portion 606 is configured generally as part of a cylindrical surface. An opposite, upward facing surface 616 of wall portion 606 is formed with a generally circular upstanding ring configuration which defines buzzer socket 558 (Figs. 12A & 12B).

Wall portion 606 is integrally formed with side portion 604 at an upper region of side portion 604. Side portion 604 is integrally formed at a lower region thereof with bottom portion 602. Bottom portion 602 preferably includes a flexible and resilient support portion 620 which is trifurcated to provide relatively independently flexible and resilient support to sensor 537 and pad 539.

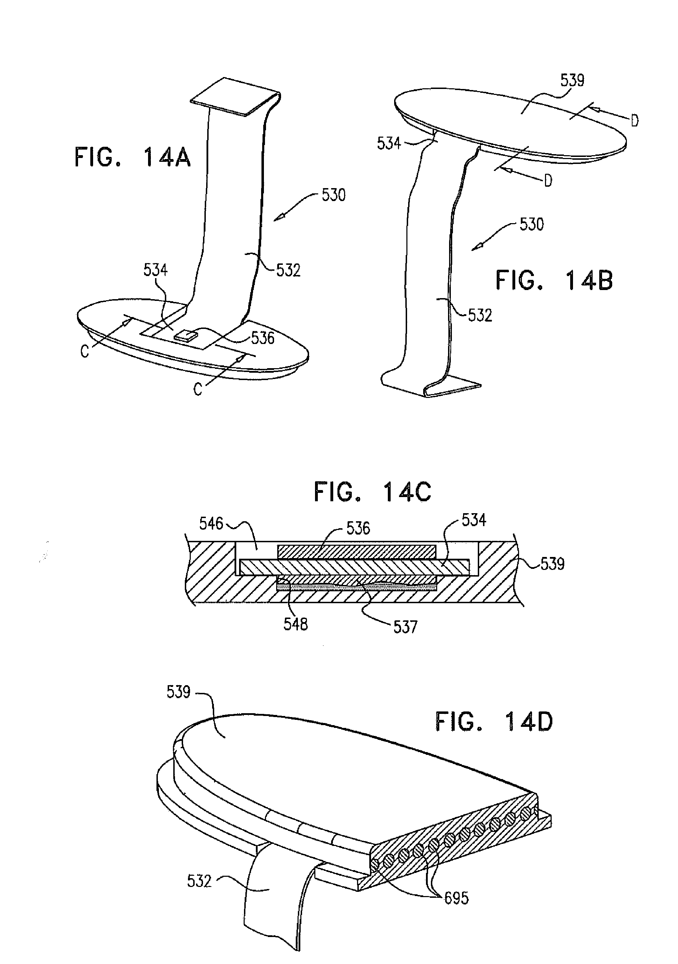

Reference is now made to Figs. 14A and 14B which are pictorial illustrations of sensor assembly 530, taken along different directions, to Fig. 14C which is a sectional illustration, taken along lines C - C in Fig. 14A, and to Fig. 14D, which is a sectional illustration of the sensor assembly of Figs. 14A and 14B, taken along lines D - D in Fig. 14B. As seen in Figs. 14A-14D, the sensor assembly 530 includes a flexible circuit connection portion 532, which defines a flexible sensor mounting portion 534, integrally formed therewith.

Top facing sensor 536 is preferably SMT mounted on top surface of portion 534, and a corresponding bottom facing sensor 537 is mounted on a bottom surface of portion 534, generally opposite sensor 536. As seen particularly clearly in Figs. 14A and 14C, sensor 537 is preferably adhesively attached to a temperature averaging skin surface contact pad 539 at a recess 548 formed therein, which recess also preferably accommodates portion 534 and sensor 536. As seen in Fig. 14D, lateral heat conducting copper elements 695 are embedded within surface contact pad 539 to provide anisotropic lateral thermal conductivity throughout the length of pad 539, which conductivity is significantly greater than the transverse thermal conductivity across the thickness of pad 539.

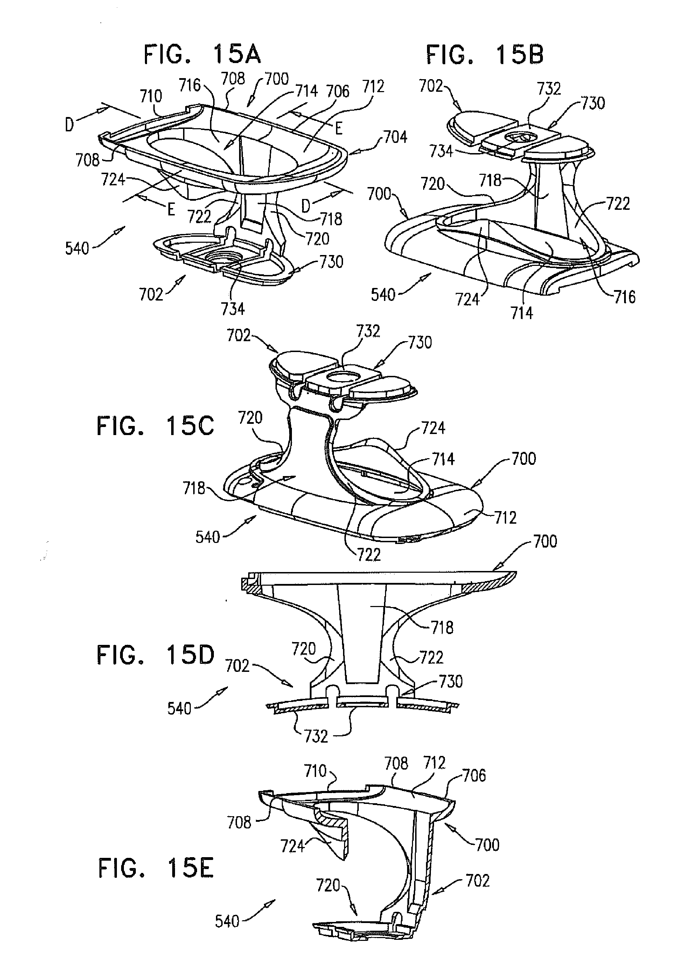

Reference is now made to Figs. 15A and 15B, which are simplified pictorial illustrations of exterior structural element 540 taken in respective downward and upward facing directions, to Fig. 15C which is a simplified pictorial illustration corresponding to Fig. 15A but taken in a generally opposite direction, to Fig. 15D, which is a simplified sectional illustration, taken along lines D - D in Figs. 15A, 15B and 15C, and to Fig. 15E, which is a simplified sectional illustration, taken along lines E - E in Figs. 15A, 15B and 15C.

As seen in Figs. 15A - 15E, exterior structural element 540 preferably includes a top portion 700 and a bottom portion 702. Top portion 700 and bottom portion 702 are preferably integrally joined.

Top portion 700 preferably includes a circumferential rim 704 which includes a main rim portion 706, two secondary side rim portions 708, which are slightly recessed with respect to the main rim portion 706 and an end rim portion 710, which is recessed with respect to side rim portions 708. A circumferential wall 712 extends between rim portions 706, 708 and 710 and about a central, generally oval aperture 714, which is, in turn, surrounded by a circumferential wall 716 extending downwardly from circumferential wall 712 in the sense of Fig. 15A.

Circumferential wall 716 includes a tapered and necked back portion 718 which is joined to bottom portion 702, side portions 720 and 722 on either side of back portion 718 and a forward lipped portion 724, seen particularly clearly in Fig. 15B.

Bottom portion 702 preferably includes a flexible and resilient support portion 730 which provides relatively independently flexible and resilient support to a flexible and resilient temperature averaging pad socket 732, including an aperture 734 which accommodates temperature averaging skin surface contact pad 539.

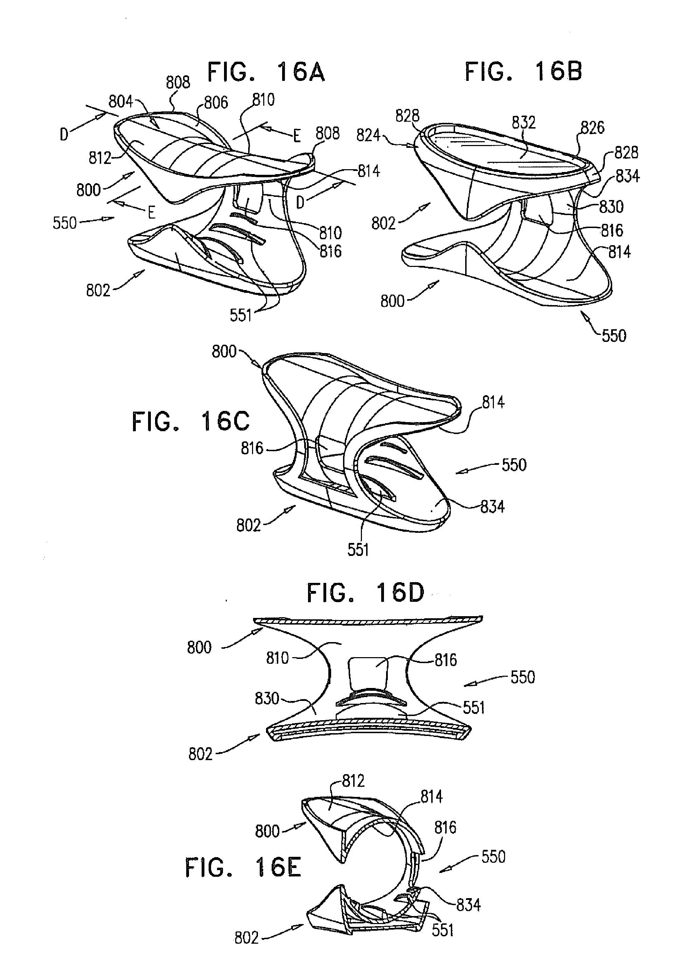

Reference is now made to Figs. 16A and 16B which are simplified pictorial illustrations of finger engagement ring element 550, which is overmolded over the assembled interior structural element 520, sensor assembly 530 and exterior structural element 540, as well as the periphery of temperature averaging pad 539, taken in respective downward and upward facing directions, to Fig. 16C which is a simplified pictorial illustration corresponding to Fig. 16A but taken in a generally opposite direction, to Fig. 16D, which is a simplified sectional illustration, taken along lines D - D in Figs. 16A, 16B and 16C, and to Fig. 16E, which is a simplified sectional illustration, taken along lines E - E in Figs. 16A, 16B and 16C.

As seen in Figs. 16A - 16E, finger engagement ring element 550 preferably includes a top portion 800 and a bottom portion 802. Top portion 800 and bottom portion 802 are preferably integrally joined.

Top portion 800 preferably includes a circumferential wall 804 which includes a lipped rim portion 806, which is seen clearly in Fig. 16A, two secondary side rim portions 808 and a side wall portion 810. Circumferential wall 804 surrounds an interior wall 812. A downward facing finger engaging surface portion 814 of wall 812 is configured generally as part of a cylindrical surface. A plurality of generally radially inwardly directly flexible resilient ribs 551 (Figs. 12A & 12B) may be integrally formed with surface 814.

Side wall portion 810 is preferably tapered and necked and is joined to bottom portion 802, a generally square cut out 816 being defined generally at this junction.

Bottom portion 802 preferably includes a circumferential wall 824 which includes a lipped portion 826, which is seen clearly in Fig. 16B, two secondary side portions 828 and a side wall portion 830. Circumferential wall 824 surrounds an interior portion 832. Interior portion 832 surrounds flexible sensor mounting portion 534, sensors 536 and 537, skin contact plate 538, and the periphery of temperature averaging skin surface contact pad 539.

Radially interiorly of flexible sensor mounting portion 534, sensors 536 and 537, skin contact plate 538 and the periphery of temperature averaging skin surface contact pad 539, interior portion 832 includes an upward facing finger engaging surface portion 834, which is configured generally as part of a cylindrical surface.

Side wall portion 830 is preferably tapered and necked and is joined to side wall portion 810 of top portion 800.

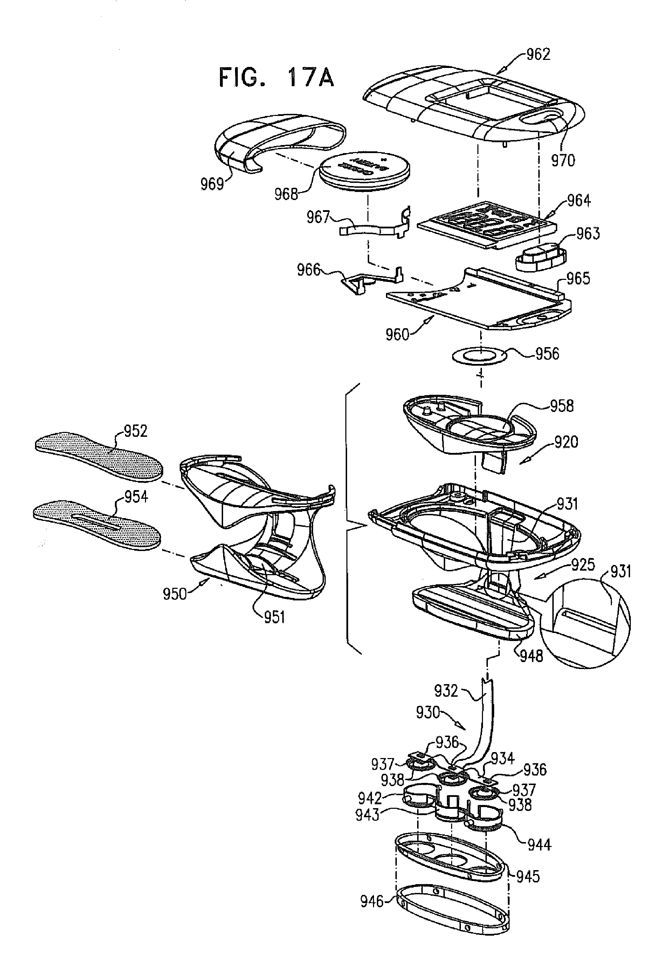

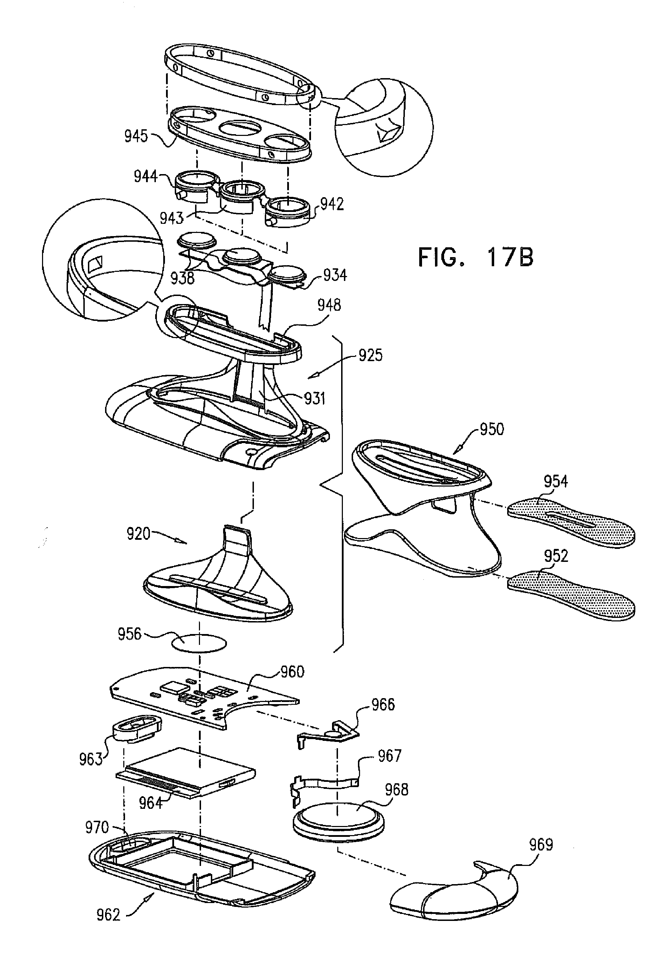

Reference is now made to Figs. 17A and 17B which are simplified exploded pictorial illustrations of yet another embodiment of a thermometer constructed and operative in accordance with a preferred embodiment of the present invention for measuring temperature of a patient, taken respectively from the top and from the bottom. As seen in Figs. 17A & 17B, the thermometer 900 comprises an interior structural element 920, typically integrally injection molded of, for example, TECHNYL® A 205F, commercially available from Rhodia Engineering Plastics SA of Paris, France, which is located within an exterior structural element 925 together with a sensor assembly 930. Elements 920, 925 and sensor assembly 930 are preferably lightly snap fitted together. Exterior structural element 925 includes a recess 931 which is provided to accommodate part of flexible circuit connection portion 930.

Sensor assembly 930 is mounted rearwardly of and below interior structural element 920, in the sense of Fig. 17A and includes a flexible circuit connection portion 932, and a flexible sensor mounting portion 934. A plurality of top facing sensors 936, preferably three in number, are mounted on a top surface of sensor mounting portion 934, and a corresponding plurality of bottom facing sensors 937 are mounted on an underneath surface of sensor mounting portion 934, generally opposite sensors 936. Sensors 936 and 937 are electrically connected to sensor mounting portion 934. A plurality of skin contact plates 938 corresponding to sensors 937 are preferably thermally adhesively mounted onto sensors 937. In alternative embodiments, top facing sensors 936 are obviated.

A first contact plate housing element 942, a center contact plate housing element 943 and a last contact plate housing element 944 are preferably overmolded by engagement surface 945 which is preferably made of silicon rubber, and are retained within a generally oval shaped contact plate housing elements retaining ring 946. As seen particularly in Fig, 17B, elements 942, 943, 944, 945 and 946 together are preferably snap fitted into a sensor supporting portion 948 of exterior structural element 925.

A finger engagement ring element 950, preferably made of TPU such as the EMPILON® HA SERIES commercially available from EMPILON® of Taiwan, is overmolded over the assembled interior structural element 920 and exterior structural element 925. The overmolding locks elements 920, 925 and 950 together.

A plurality of generally radially inwardly directly flexible resilient ribs 951 may be integrally formed as part of finger engagement ring element 950 for low thermal conductivity finger engagement with fingers of various sizes. Resilient finger engagement pads 952 and 954 are optionally provided for adapting the fit of finger engagement ring element 950 to various finger sizes. Pads 952 and 954 preferably are formed of a good thermal insulator such as closed cell foam so as to provide good thermal insulation between a user's finger and sensors.

A buzzer 956 is preferably seated in a buzzer socket 958 formed on interior structural element 920.

A printed circuit board 960 is mounted onto a top cover panel 962 over exterior structural element 925. Various electrical components are mounted onto printed circuit board 960 including, inter alia, an on-off push button 963, an LCD display 964, an LCD connector 965 and battery mounting brackets 966 and 967. A battery 968 is mounted on brackets 966 and 967 and is accessed via a slidably removable battery cover 969. Top cover panel 962 is mounted onto exterior structural element 925 to enclose printed circuit board 960, LCD display 964, LCD connector 965 as well as bracket 967 and part of battery 968. Top cover panel 962 is preferably formed with an aperture 970 for accommodating push button 963, which protrudes therethrough.

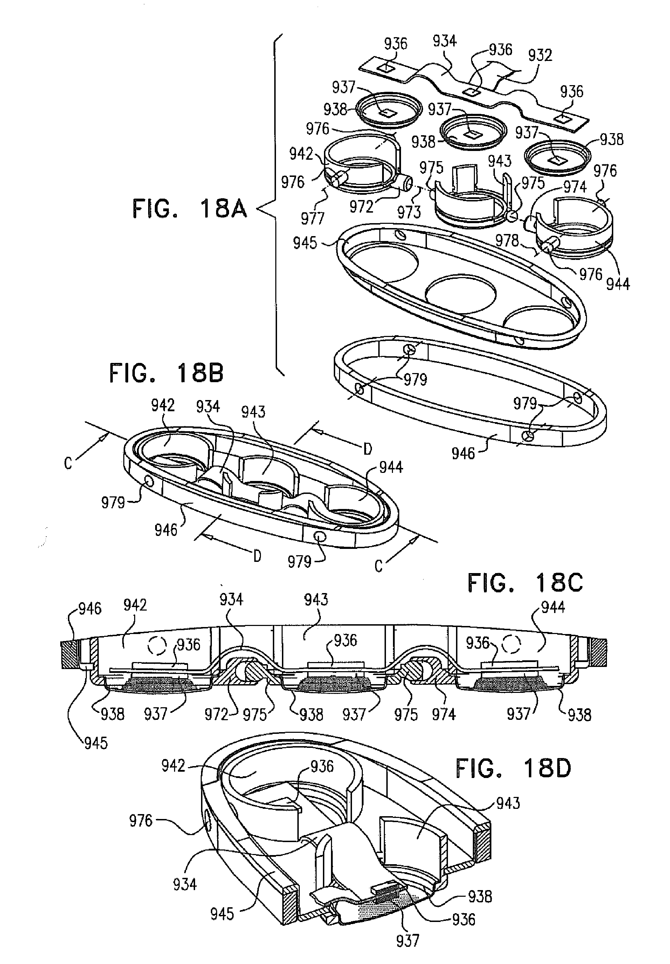

Reference is now made to Figs. 18A and 18B, which are pictorial illustrations of a sensor assembly, forming part of the thermometer of Figs. 17A and 17B, and to Fig. 18C which is a sectional illustration of the sensor assembly of Figs. 18A and 18B, taken along lines C - C in Fig. 18B. As seen in Figs. 18A - 18C, the sensor assembly 930 includes a flexible circuit connection portion 932 and a flexible sensor mounting portion 934 extending generally perpendicular to portion 932 and integrally formed therewith.

A plurality of top facing sensors 936, preferably three in number, are preferably SMT mounted on a top surface of sensor mounting portion 934, and a corresponding plurality of bottom facing sensors 937 are preferably SMT mounted on an underneath surface of sensor mounting portion 934, generally opposite sensors 936. Sensors 936 and 937 are electrically connected to sensor mounting portion 934. In alternative embodiments, top facing sensors 936 are obviated.

As seen particularly clearly in Fig. 18C, skin contact plates 938 are preferably adhesively attached to sensors 937. Skin contact plates 938 are preferably formed of a heat conductive material such as metal, such as stainless steel 304 and are joined to sensors 937 by a highly thermally conductive adhesive such as Robnor Glue #PX439XS, commercially available from Robnor Resins Ltd. of Swindon, United Kingdom.

A first contact plate housing element 942, a center contact plate housing element 943 and a last contact plate housing element 944 are preferably overmolded by engagement surface 945 and are retained within a generally oval shaped contact plate housing elements retaining ring 946.

As seen in Figs. 18A and 18C, element 942 includes a joint socket 972 protruding along an axis 973 facing element 943, and element 944 includes a joint socket 974 protruding along axis 973 facing element 943, generally opposite joint socket 972. Element 943 includes two joint balls 975 which protrude from opposite sides of element 943 along axis 973, and which interconnect with joint sockets 972 and 974 to form two ball and joint sockets which flexibly connect element 943 to element 942 and element 943 to element 944, respectively.

Each of elements 942 and 944 also include two oppositely protruding pins 976 which are arranged along axis 977 and 978 respectively, whereby axis 977 and 978 are generally perpendicular to axis 973. Pins 976 protrude through a rim of overmolded engagement surface 945 and are loosely and rotatably disposed within holes 979 located in the perimeter of retaining ring 946, allowing for partial rotation of elements 942 and 944 about axis 977 and 978 respectively. This arrangement of elements 942, 943 and 944 within retaining ring 946 provides for sufficient flexibility so as to be adapted for temperature sensing touching engagement with either a generally convex body portion, or a generally concave body portion.

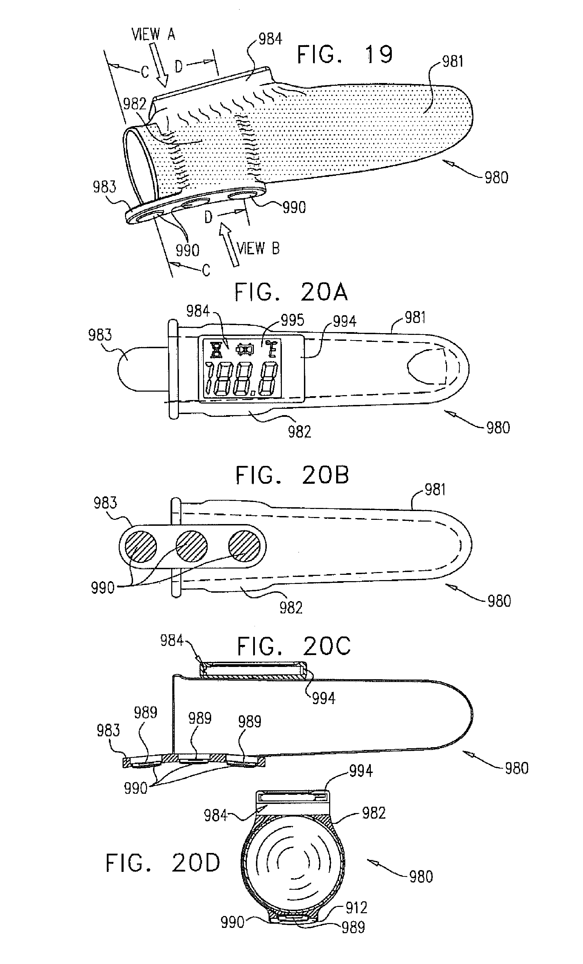

Reference is now made to Fig. 19 and to Figs. 20A, 20B, 20C and 20D, which are respectively a simplified pictorial illustration of a thermometer constructed and operative in accordance with another preferred embodiment of the present invention for measuring temperature of a patient, and simplified side view illustrations taken along directions indicated by arrows A and B in Fig. 19 and simplified sectional illustrations taken along respective lines C - C and D - D in Fig. 19.

As seen in Figs. 19-20D, there is provided a finger surrounding thermometer 980 including a finger cot type element 981, which is adapted to be removably mounted onto a user's finger. Finger cot type element 981 may be formed of any suitable material, such as latex, rubber, plastic or cotton.

A ring subassembly 982 is mounted onto finger cot type element 981 and includes a body surface curvature and finger bending responsive engagement and temperature sensing portion 983 arranged to be located facing opposite one finger portion, and a user interface and temperature display portion 984 arranged to be located facing opposite another finger portion, which located generally on the other side of the finger from the engagement and temperature sensing portion 983.

It is a particular feature of the present invention that the body surface curvature responsive and finger bending responsive engagement and temperature sensing portion 983 is sufficiently flexible and resilient so as to be adapted for temperature sensing touching engagement with a generally convex body portion, such as a forehead, and with a generally concave body portion, such as an armpit.

Reference is now made particularly to Figs. 20C and 20D, which illustrate in section, the internal structure of the thermometer 980. As seen particularly in Figs. 20C & 20D, the thermometer 980 comprises a body surface curvature and finger bending responsive engagement and temperature sensing portion 983 and a plurality of sensors 989, preferably three in number, mounted on portion 983 and electrically connected thereto, and a corresponding plurality of skin contact plates 990, preferably thermally adhesively mounted onto sensors 989.

It is a further particular feature of the present invention that in addition to the overall resiliency and flexibility of the body surface curvature responsive and finger bending responsive engagement and temperature sensing portion 983, individual sensors 989 are mutually independently mounted in body surface curvature responsive and finger bending responsive engagement and temperature sensing portion 983, so as each to be independently arrangeable in a spatial orientation which may be independently different from the spatial orientation of each other of said plurality of sensors.

A printed circuit board (not shown) is mounted onto a top cover panel 994 over an exterior structural element (not shown). Various electrical components are mounted between the printed circuit board and top cover panel 994 including, inter alia, an LCD display 995 as well as an on-aff push button, an LCD connector, a battery and battery mounting brackets (all not shown). Top cover panel 994 is preferably formed with an aperture (not shown) to accommodate the push button, which protrudes therethrough.

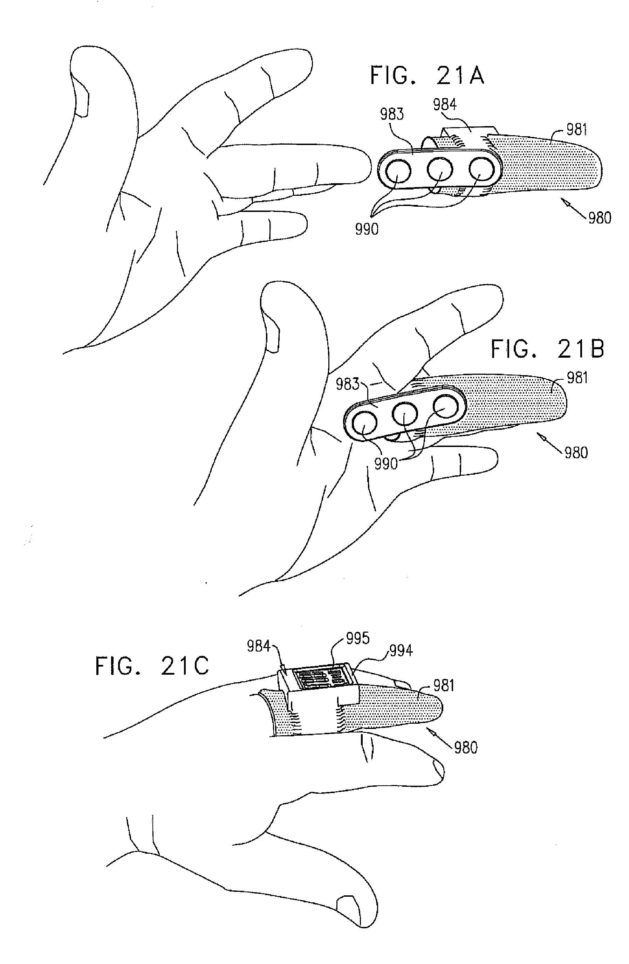

Figs. 21A, 21B and 21C show that the thermometer 980 may be engaged with a finger of a person measuring temperature as a finger cot is slipped onto the finger. Preferably, the ring subassembly 982 sits on the proximal phalange of the finger as shown. It is preferred that the sensors 989 be arranged with respect to the remainder of the thermometer 980 in a symmetric arrangement such that the thermometer is ambidextrous.

It is appreciated that the sensors in all of the aforementioned embodiments are operative to provide sensor temperature readings which can be used to calculate a local deep tissue temperature which reflects a temperature at a location under the skin that is the source of the heat conducted to the sensors. The local deep tissue temperature readings are then used to calculate a core body temperature. The calculation of the local deep tissue temperature and of the core body temperature can be achieved in a single calculation using the formulas described in US Patent 6,280,397 (col. 2 lines 15 - 49) and in US Patent 7,597,668 (col. 9, lines 31 - 67), both of the applicant/assignee and incorporated by reference hereinabove.

Alternatively, a predicted temperature, equivalent to the local deep tissue temperature, can be calculated using a predictive algorithm as in US Patent 4,648,055 (col. 13, line 20) by Ishizaka et al. or as in US Patent 5,738,441 (col. 13, line 25) by Cambridge et al, After calculating the local deep tissue temperature, the correction for the core body temperature can be calculated using the following empirical formula:

$$T_{core} = a1 * T_{ldt} + a2 * T_{ldt}^{2} + a3 * T_{ldt}^{3} + a4$$

where:

$T_{core}$ is the core body temperature;
$a1$, $a2$, $a3$ and $a4$ are constants determined by experiment; and
$T_{ldt}$ is the local deep tissue temperature, equivalent to the predicted temperature calculated in US Patents 4,648,055 and 5,738,441.

It is noted that the temperature of the environment, or the ambient temperature, affects the sensor temperature readings. Therefore, the calculated local deep tissue temperature must be corrected to account for the ambient temperature. The necessary correction can be calculated using the following empirical formula:

$$T_{ldt\_corrected} = T_{ldt} + \alpha * (25 - T_{amb}) + \eta$$

where:

$T_{ldt\_corrected}$ is the local temperature, corrected to account for the ambient temperature;
$T_{ldt}$ is the originally calculated local deep tissue temperature, without the correction to account for the ambient temperature;
$T_{amb}$ is the ambient temperature; and
$\alpha$ and $\eta$ are constants determined by experiment.

Alternatively, the correction for the ambient temperature may take into account the difference between the local deep tissue temperature and the ambient temperature as follows:

$$T_{ldt\_corrected} = T_{ldt} + (T_{ldt} - T_{amb}) * \beta + \gamma$$

where:

$T_{ldt\_corrected}$ is the local temperature, corrected to account for the ambient temperature;

$T_{ldt}$ is the originally calculated local deep tissue temperature, before correction to account for the ambient temperature;
$T_{anth}$ is the ambient temperature; and
β and γ are constants determined by experiment.

The ambient temperature is recorded by the thermometer before applying the thermometer to the skin surface.
When measuring temperature at a location on the skin in the vicinity of blood vessels, temperature readings may vary with the distance between the thermometer and the blood vessels. At the center of the forehead, this deviation is typically about 1.2 degrees Celsius per every 10 millimeters of lateral distance. It is therefore useful to employ a sensor array as shown in US published patent application 2009/0299682 incorporated by reference herein.
The calculation of the local deep tissue temperature comprises calculating an average, a weighted average, or any other function which of the temperature readings received from each of the sensors in the sensor array. This averaging is useful in overcoming the effect of temperature readings which vary with the distance between the thermometer and the blood vessels.
Additionally, the core body temperature can be calculated based solely on local deep tissue temperature readings obtained from three sensors of the array using the following formula:

$$T_{core} = a1 * T_{\max} + a2 * T_{mid} + a3 * T_{\min} + a4$$

where :

$T_{core}$ is the core body temperature;
$a1, a2,$ a3 and a4 are constants determined by experiment; and
$T_{\max}$, $T_{mid}$ and $T_{\min}$ are the minimum, intermediate and maximum local deep tissue temperature readings of the 3 sensors, which may be corrected to account for the ambient temperature, as can be calculated using the equations for $T_{ldt\_corrected}$ as shown hereinabove.

It will be appreciated by persons skilled in the art that the present invention is not limited by what has been particularly shown and described hereinabove. Rather the scope of the present invention includes both combinations and subcombinations of the various features described hereinabove as well as modifications thereof which would occur to persons skilled in the art upon reading the foregoing description and which are not in the prior art.

**Claims**

1. A thermometer useful for measuring the temperature of a patient, the thermometer comprising:

   a housing;
   at least one temperature sensor assembly including at least one external patient skin contacting surface thermally connected to at least one temperature detector in a manner that the temperature sensed at said at least one temperature detector generally represents an average of temperatures at said at least one external patient skin contacting surface; and
   a mounting element for mounting said at least one temperature sensor assembly on said housing.

2. A thermometer according to claim 1 and wherein
   said mounting element for mounting said at least one temperature sensor assembly on said housing is operative for mutually independently mounting each of a plurality of said at least one external patient skin contacting surface to be independently arrangeable in a spatial orientation which may be independently different from the spatial orientation of the external patient skin contacting surface of each other of said plurality of said at least one external patient skin contacting surface.

3. A thermometer according to claim 2 and wherein each of said at least one temperature sensor assembly includes a plurality of temperature detectors, each temperature detector being associated with one of said plurality of external patient skin contacting surfaces.

4. A thermometer according to claim 3 and wherein the temperature sensed at each of said plurality of temperature detectors generally represents an average of temperatures at said plurality of external patient skin contacting surfaces.

5.  A thermometer according to claim 1 and wherein said thermometer also comprises a user interface and temperature display portion.

6.  A thermometer according to claim 2 and wherein said plurality of external patient skin contacting surfaces are independently arrangeable in response to at least one of contact with a patient skin surface and bending of a user appendage onto which said housing is mounted.

7.  A thermometer according to claim 6 and wherein:

    said at least one temperature sensor assembly is arranged to be facing in a first direction away from said user appendage; and
    said user interface and temperature display portion is arranged to be facing away from said user appendage in a second direction, generally opposite to said first direction; and
    said housing comprises an at least partially closed ring portion supporting said at least one temperature sensor assembly and said user interface and temperature display portion for mounting onto said user appendage.

8.  A thermometer according to claim 7 and wherein at least one of said housing and said mounting element is adapted to provide thermal insulation between said at least one temperature sensor assembly and a user's appendage.

9.  A thermometer according to claim 7 and wherein said at least partially closed ring portion is mountable on the finger of a user.

10. A thermometer according to claim 1 and wherein at least one of said housing and said mounting element provides thermal insulation between individual ones of said external patient skin contacting surfaces.

11. A thermometer according to claim 1 and wherein said housing is ambidextrous.

12. A thermometer according to claim 1 and wherein said housing comprises a finger cot.

13. A thermometer according to claim 1 and wherein said mounting element is adapted to adopt a curvature which is responsive to a curvature of a patient body surface engaged thereby and to said flexing of a user appendage onto which said housing is mounted.

14. A thermometer according to claim 1 and wherein said housing is adapted to accommodate various different appendage sizes.

15. A thermometer according to claim 1 and wherein said housing is stretchable.

FIG. 1

FIG. 2

## FIG. 3A

## FIG. 3B

FIG. 4A

FIG. 4B

## FIG. 5A

## FIG. 5B

## FIG. 5C

## FIG. 5D

## FIG. 5E

FIG. 6A

130

132

136

136

136

137

138

134

138

137

138

C

C

137

FIG. 6B

138

134

130

132

FIG. 6C

137　136

134

138

FIG. 7A

FIG. 7B

FIG. 7C

FIG. 7D

FIG. 7E

FIG. 8A

FIG. 8B

FIG. 8C

FIG. 8D

FIG. 8E

174    162   164
100                              C

C
312
FIG. 9A        151
                              VIEW-B
                        154
163

162        163
100

312
FIG. 9B        152

151

154

174
100            162
                   164
312            160
140            152
150        410   416
FIG. 9C    151   430
150            154
140   120

FIG. 10A

FIG. 10B

FIG. 10C

FIG. 11A

FIG. 11B

FIG. 11C

FIG. 12A

FIG. 12B

FIG. 13A

FIG. 13B

FIG. 13C

FIG. 13D

FIG. 13E

FIG. 14A

FIG. 14B

530

539

534

534

536

532

532

D

D

530

C

C

FIG. 14C

546

536

534

539

548

537

FIG. 14D

539

532

695

FIG. 15A

FIG. 15B

FIG. 15C

FIG. 15D

FIG. 15E

## FIG. 16A

## FIG. 16B

## FIG. 16C

## FIG. 16D

## FIG. 16E

FIG. 17A

FIG. 17B

FIG. 18A

936  934  936  932
937
938  937
976  938
942  975  943
976  977  972  973  975  974  976
945  978  976  944

979

979

FIG. 18B

942  934  943
C  D
944
979
946  979  C
D

FIG. 18C

946  942  934  943
936  936  936
944
938  937  972  975  938  937  975  974  937  938
945

FIG. 18D

942  936
976  943
934
945  938
937  936

FIG. 19

FIG. 20A

FIG. 20B

FIG. 20C

FIG. 20D

FIG. 21A

FIG. 21B

FIG. 21C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2658390 A
- US 3273395 A
- US 3282106 A
- US 3374354 A
- US 3392282 A
- US 3491596 A
- US 3581570 A
- US 3597976 A
- US 3614892 A
- US 3719838 A
- US 3777568 A
- US 3781837 A
- US 3933045 A
- US 4005605 A
- US 4133700 A
- US 4141149 A
- US 4198861 A
- US 4204120 A
- US 4302971 A
- US 4317998 A
- US 4372690 A
- US 4420265 A
- US 4428382 A
- US 4456390 A
- US 4494881 A
- US 4566808 A
- US 4602642 A
- US 4623266 A
- US 4626686 A
- US 4634294 A
- US 4636091 A
- US 4691713 A
- US 4722612 A
- US 4726688 A
- US 4784149 A
- US 4790324 A
- US 4797840 A
- US 4831258 A
- US 4843577 A
- US 4846583 A
- US 4895164 A
- US 4932789 A
- US 4993419 A
- US 5012813 A
- US 5017019 A
- US 5018872 A
- US 5024533 A
- US 5050612 A
- US 5056929 A
- US 5150969 A
- US 5159936 A
- US 5167235 A
- US 5178464 A
- US 5187943 A
- US 5199436 A
- US 5229612 A
- US 5232284 A
- US 5271407 A
- US 5325863 A
- US 5333784 A
- US 5368038 A
- US 5381796 A
- US 5441476 A
- US 5445158 A
- US 5469855 A
- US RE35554 E
- US 5645349 A
- US 5653238 A
- US 5695283 A
- US 5743644 A
- US 5816706 A
- US 5818044 A
- US 5872362 A
- US 5874736 A
- US 5893833 A
- US 5924996 A
- US 5924998 A
- US 6047205 A
- US 6056435 A
- US 6059452 A
- US 6086247 A
- US 6090050 A
- US 6220750 B
- US 6280397 B
- US 6292685 B
- US 6299347 B
- US 6315719 B
- US 6416471 B
- US 6447460 B
- US 6499877 B
- US 6547745 B
- US 6646567 B
- US 6709154 B
- US 6847913 B
- US 6890096 B
- US 6898457 B
- US 6929611 B
- US 6932775 B

- US 6963772 B
- US 7213969 B
- US 7340293 B
- US 7346386 B
- US 7479116 B
- US 7597668 B
- US 7625117 B
- US 20010044588 A
- US 20010047127 A
- US 20020143257 A
- US 20030149349 A
- US 20030173408 A
- US 20040170216 A
- US 20050245839 A
- US 20060047218 A
- US 20060161074 A
- US 20060173375 A
- US 20060264730 A
- US 20070100666 A
- US 20080008225 A
- US 20090299682 A
- DE 1914468
- EP 0411121 A
- EP 0446788 A
- GB 1226540 A
- JP 55037917 B
- WO 8100764 A
- WO 8606163 A
- WO 9303666 A
- WO 9801730 A
- WO 9808431 A
- WO 9939166 A
- US 4648055 A
- US 5738441 A, Ishizaka

**Non-patent literature cited in the description**

- Surface Temperature Scanner STS-100-F/C & 101-C. Omega Medical Corporation, 1987, 2
- Sensor Touch Temporal Artery Thermometer--The Noninvasive Thermometer You Can Trust. Exergen Corporation, 1999, 5
- WEI, D. et al. *Optimal Design of a Thermistor Probe for Surface Measurement of Cerebral Blood Flow, Transactions on Biomedical Engineering,* 1990, vol. 37 (12), 1159-1172
- Database WPI. Derwent Publications Ltd, 15 May 1994
- FirstTemp Intelligent Medical Systems, Model 2000A, Operation Manual. 8
- ConforTemp All-Family Instant Underarm Thermometer. *Quick Start Instructions,* 6
- FirstTemp Clinical Thermometer, Technical Manual. 24
- *Omega Medical Surface Temperature Scanner STS-100-F/C & 101-C,* 2
- DET TRONICS, Optical Calibration. October 1987
- Model 1M Thermopile Detector. Dexter Research Center, October 1980
- FRADEN ; JACOB. Application of Piezo/Pyroelectric Films in Medical Transducers. *J. Clinical Engineering,* 1988, vol. 13 (3), 133-138
- HOUDAS, Y. ; RING, E.F.J. Human Body Temperature, Its Measurement and Regulation. Plenum Press, 1982, 83
- LOONEY, JR. ; JOSEPH M. ; POMPEI, FRANCISCO. Ear Thermometry. Medical Electronics, June 1989
- Surface Temperature Scanner, Models: STS100-F/C, STS-101-C, User Manual. 9
- *Proceedings of the Eighth Annual Conference of the IEEE/Engineering in Medicine and Biology Society,* 07 November 1986, vol. 3
- RYAN ; MARYBETH. The Equine Infrared Thermographic Scanner: Assuring Performance of the Equine Athlete. Exergen Corporation, 1989, 4
- POMPEI ; MARYBETH. Temperature Assessment via the Temporal Artery: Validation of a New Method. Exergen Corporation, 1999
- KUZUCU, ETHAM Y. Measurement of Temperature. *Int. Anesthesiol Clin.,* 1965, vol. 3 (3), 435-449
- PET.TM. Physician's Electronic Thermometer. Phillips Bio-Medical, Inc, 2
- Non-invasive temporal artery thermometry: Physics, physiology, and clinical accuracy. POMPEI ; M. POMPEI. Proceedings of SPIE. April 2004, vol. 5405, 61-67
- D. FIALA ; K. J. LOMAS ; M. STOHRER. A computer model of human thermoregulation for a wide range of environmental conditions: the passive system. *J Appl Physiol,* 1999, vol. 87 (8750-7587), 1957-1972
- Y. H. CHIOK ; E. Y.-K. NG ; V. V. KULISH. Global bioheat model for quick evaluation of the human physiological thermal profiles under differing conditions. *J Med Eng Technol,* 2002, vol. 26, 231-238
- Z.-S. DENG ; J. LIU. Mathematical modeling of temperature mapping over skin surface and its implementation in thermal disease diagnostics. *Comput Biol Med,* 2004, vol. 34, 495-521
- S. B. WILSON ; V. A. SPENCE. A tissue heat transfer model for relating dynamic skin temperature changes to physiological parameters. *Phys Med Biol,* 1988, vol. 33, 895-912